# FASCICULE DE BREVET EUROPEEN

(11) **EP 1 027 881 B1**
(45) Date de publication et mention de la délivrance du brevet: **07.09.2005**
(21) Numéro de dépôt: 00400244.0
(22) Date de dépôt: 31.01.2000
(51) Int. Cl.: A61K 7/42

(54) **Compositions photoprotectrices contenant un dérivé de bis-résorcinyl triazine et un composé à groupements benzoazolyle ou benzodiazolyle**
Lichtschutzzusammensetzungen enthaltend eines Bisresorcinyltriazinderivat und eine Verbindung mit Benzoazolyl- oder Benzodiazolylgruppen
Photoprotective compositions containing a bisresorcinyl triazine derivative and a compound with benzoazolyl or benzodiazolyl groups

(30) Priorité: 12.02.1999 FR 9901732
(43) Date de publication de la demande: 16.08.2000
(73) Titulaire: L'OREAL, 75008 Paris (FR)
(72) Inventeur: Candau, Didier, 91570 Bievres (FR)
(74) Mandataire: Miszputen, Laurent

(56) Documents cités:
- EP-A- 0 669 323
- EP-A- 0 775 698
- EP-A- 0 824 909
- EP-A- 0 878 469
- CH-A- 350 763
- DE-A- 19 548 014

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre (a) au moins un dérivé bis-résorcinyl triazine à titre de premier filtre et (b) au moins un composé comportant au moins deux groupes benzoazolyle ou un composé comportant au moins un groupe benzodiazolyle à titre de deuxième filtre ; lesdits premier et second filtres étant présents dans lesdites compositions dans une proportion produisant une activité synergique au niveau des indices de protection conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est-à-dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison de deux filtres solaires particuliers et déjà connus en soi dans l'état de l'art, permettait, du fait d'un effet de synergie remarquable, d'obtenir des compositions antisolaires présentant des indices de protection nettement améliorés.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (a) au moins un dérivé bis-résorcinyl triazine à titre de premier filtre et (b) au moins un composé comportant au moins deux groupes benzoazolyle ou un composé comportant au moins un groupe benzodiazolyle à titre de deuxième filtre ; lesdits premier et second filtres étant présents dans lesdites compositions dans une proportion produisant une activité synergique au niveau des indices de protection conférés.

La présente invention a également pour objet l'utilisation de telles compositions pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

Ainsi, conformément à l'un des objets de la présente invention, il est maintenant proposé de nouvelles compositions cosmétiques ou dermatologiques, en particulier antisolaires, qui sont essentiellement caractérisées par le fait qu'elles comprennent, dans un support cosmétiquement acceptable :
(a) au moins un dérivé bis-résorcinyl triazine à titre de premier filtre et
(b) au moins un composé comportant au moins deux groupes benzoazolyle ou un composé comportant au moins un groupe benzodiazolyle à titre de deuxième filtre ; lesdits premier et second filtres étant présents dans lesdites compositions dans une proportion produisant une activité synergique au niveau des indices de protection conférés.

On entendra dans la description par «comportant au moins deux groupes benzoazolyle» comme comportant par molécule au moins deux groupes du type benzoxazolyle, benzothiazolyle ou benzimidazolyle.

On entendra dans la description par «comportant au moins un groupe benzodiazolyle» comme comportant par molécule un groupe du type benzodioxazolyle, benzodithiazolyle ou benzodiimidazolyle.

Les dérivés de bis-résorcinyl triazine conformes à l'invention sont de préférence choisis parmi les composés répondant à la formule suivante : 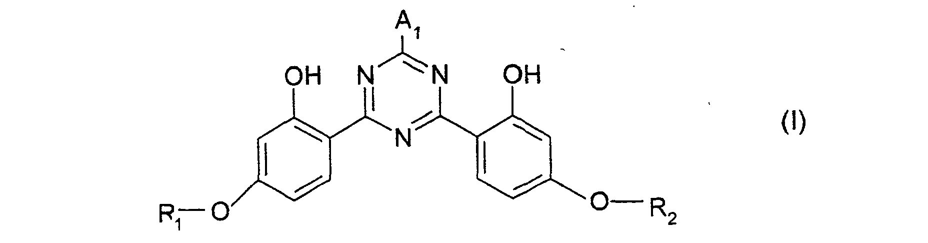 dans laquelle :
(i) les radicaux R₁ et R₂, identiques ou différents, désignent un radical alkyle en, C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ;
(ii) les radicaux R₁ et R₂, identiques ou différents, peuvent aussi désigner un reste de formule (1) suivante : 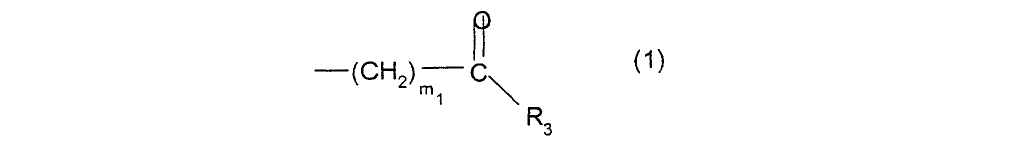 dans laquelle :
   - m₁ est un nombre de 1 à 3 ;
   - R₃ désigne un groupe hydroxy ; un radical alkyle en C₁-C₅ non substitué ou substitué par un ou plusieurs groupes hydroxy; un radical alcoxy en C₁-C₅ ; un groupe amino ; un radical mono- ou dialkyl(C₁-C₅)amino ; un cation métallique M ; un reste choisi parmi l'une des formules (2) à (7) suivantes : 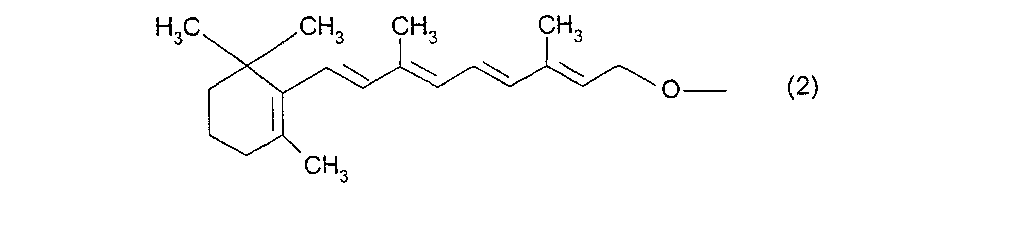 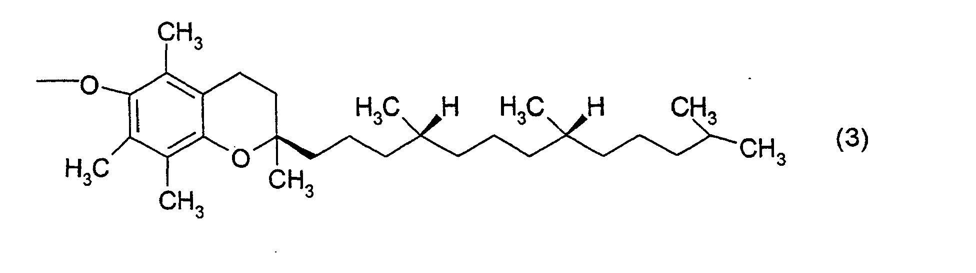 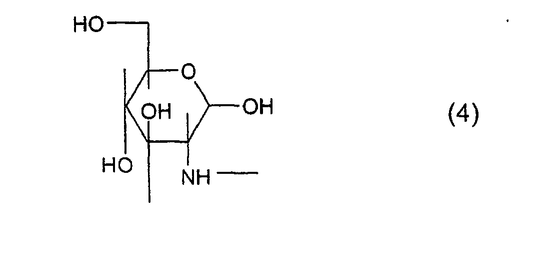 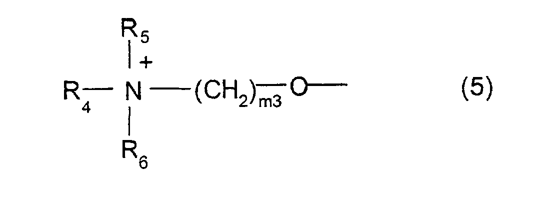 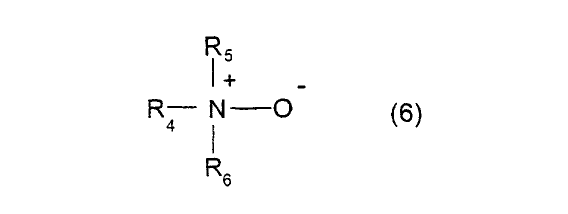 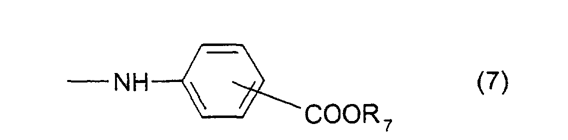
   dans lesquelles
   - les radicaux R₄, R₅ et R₆, identiques ou différents, désignent un radical alkyle en C₁-C₁₄ non substitué ou substitué par un ou plusieurs groupes hydroxy ;
   - m₃ est un nombre de 2 à 14 ;
   - R₇ représente un atome d'hydrogène, un cation métallique M, un radical alkyle en C₁-C₅ ou un reste de formule -(CH₂)ₘ₂-OT₁ où m₂ est un nombre de 1 à 4 et T₁ a la même signification indiquée ci-dessus ;
(iii) les radicaux R₁ et R₂, identiques ou différents, peuvent encore désigner un reste de formule (8) suivante : 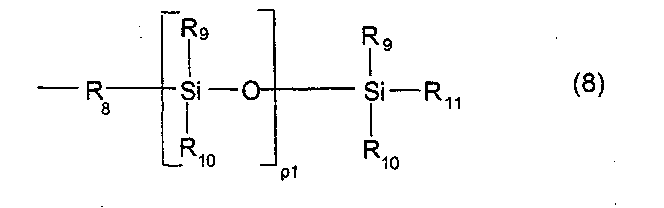 dans laquelle :
   - R₈ désigne une liaison covalente ; un radical alkyle linéaire ou ramifié en C₁-C₄ ou bien un reste de formule -Cₘ₄H₂ₘ₄- ou -Cₘ₄H₂ₘ₄-O- où m₄ est un nombre de 1 à 4 ;
   - p₁ est un nombre de 0 à 5 ;
   - les radicaux R₉, R₁₀ et R₁₁, identiques ou différents, désignent un radical alkyle en C₁-C₁₈ ; un alcoxy en C₁-C₁₈ ou un reste de formule : 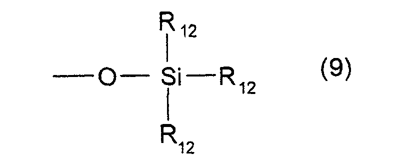
   où R₁₂ est un radical alkyle en C₁-C₅ ;
   - A₁ désigne un reste répondant à l'une des formules suivantes : 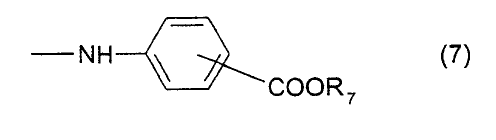 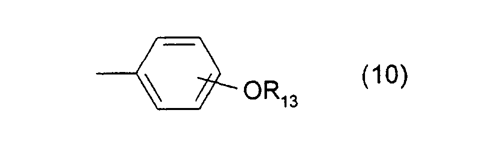 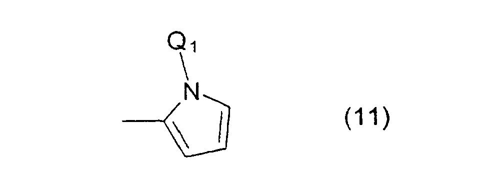 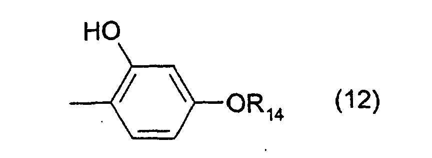
   dans lesquelles :
   - R₇ a les mêmes significations indiquées ci-dessus ;
   - R₁₃ désigne un atome d'hydrogène, un radical alkyle en C₁-C₁₀, un radical de formule : -(CH₂CHR₁₆-O)ₙ₁R₇ où n₁ est un nombre de 1 à 16, R₁₆ est hydrogène ou méthyle ou bien un reste de structure -CH₂-CH-(OH)-CH₂OT₁ avec T₁ ayant la même signification indiquée ci-dessus.
   - Q₁ est un radical alkyle en C₁-C₁₈ ;
   - R₁₄ est un radical répondant à la formule (1) : 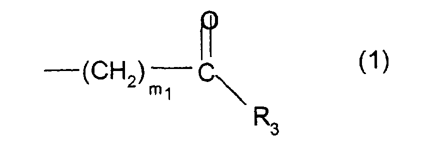
   telle que définie ci-dessus.

Dans les formules (I) et (1) à (12) décrites ci-dessus :
- les radicaux alkyle sont linéaires ou ramifiés et peuvent être choisis par exemple parmi méthyle, éthyle, n-propyle, isopropyle, n-butyle, sec.butyle, tert.butyle, amyle, isoamyle, tert.amyle, heptyle, octyle, isooctyle, nonyle, décyle, undécyle, dodécyle, tétradécyle, pentadécyle, hexadécyle, heptadécyle ou octadécyle ;
- les radicaux alcényle en C₂-C₁₈, peuvent être choisis par exemple parmi allyle, méthallyle, isopropènyle, 2-butènyle, 3-butènyle, isobutènyle, n-penta-2,4-diènyle, 3-méthyl-but-2-enyle, n-oct-2-enyle, n-dodec-2-enyle, iso-dodecenyle, n-octadec-4-enyle ;
- les radicaux alcoxy sont linéaires ou ramifiés et peuvent être choisis par exemple parmi méthoxy, éthoxy, n-propoxy, isopropoxy, n-butoxy, sec.-butoxy, tert.-butoxy, amyloxy, isoamyloxy ou tert.amyloxy ;
- les radicaux mono ou dialkylamino peuvent être choisis par exemple parmi méthylamino, éthylamino, propylamino, n-butylamino, sec.butylamino, tert.butylamino, pentylamino, diméthylamino, diéthylamino, dibutylamino ou méthyléthylamino.
- les cations métalliques M sont des cations alcalins, alcalino-terreux ou métalliques choisis par exemple parmi lithium, potassium, sodium, calcium, magnésium, cuivre et zinc.

Les dérivés de bis-résorcinyl triazine de formule (I) de l'invention sont des filtres déjà connus en soi. Ils sont décrits et préparés selon les synthèses indiquées dans les demandes de brevet EP-A-0775698 et EP-A-0878469 (faisant partie intégrante du contenu de la description).

A titre d'exemples de composés bis-résorcinyl triazine de formule (I) utilisables, on peut citer ceux pour lesquels le radical A₁ désigne para-méthoxyphényle ou 4-éthoxyphényle et les radicaux R₁ et R₂, identiques ou différents, désignent un radical de structure : 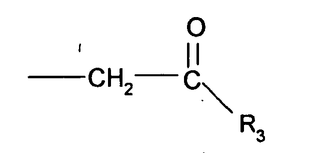 dans laquelle R₃ est choisi parmi :
- tert.butyloxy ;
- OH ;
- OM où M est un cation alcalin, alcalino-terreux ou choisi parmi le cuivre, le magnésium ou le zinc ;
- un groupe de structure : 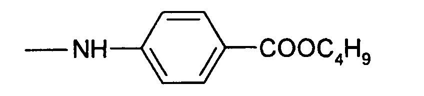
- un groupe de structure O⁻N⁺(CH₂CH₂OH)₃ ;
- un groupe de structure : 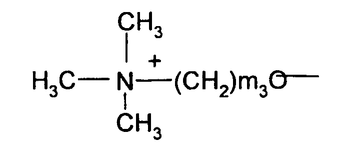 avec m₃ variant de 2 à 14;
- un groupe de structure : 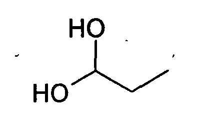
- un groupe de structure :

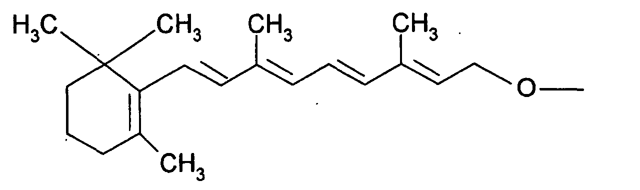

On peut également mentionner le composé bis-résorcinyl triazine répondant à la formule (I) dans laquelle le radical A₁ désigne para-hydroxyphényle et les radicaux R₁ et R₂ désignent simultanément un groupe de structure :

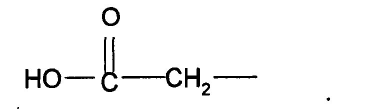

A titre d'exemples de composés de formule (I) utilisables, on peut citer :
- la 2,4-bis{[4-(2-éthylhexyloxy)-2-hydroxy]-phényl}-6-(4-méthoxy-phenyl)-1,3,5-triazine ;
- la 2,4-bis{[4-(3-(2-propyloxy)-2-hydroxy-propyloxy)-2-hydroxy]-phényl}-6-(4-méthoxyphényl)-1,3,5-triazine ;
- la 2,4-bis{[4-(2-éthyl-hexyloxy)-2-hydroxy]-phényl}-6-[4-(2-méthoxyéthylcarboxyl)-phénylamino]-1,3,5-triazine ;
- la 2,4-bis{[4-tris(triméthylsiloxy-silylpropyloxy)-2-hydroxy]-phényl}-6-(4-méthoxyphényl)-1,3,5-triazine ;
- la 2,4-bis{[4-(2''-méthylpropenyloxy)-2-hydroxy]-phényl}-6-(4-méthoxyphényl)-1,3,5-triazine ;
- la 2,4-bis{[4-(1',1',1',3',5',5',5'-heptaméthyltrisiloxy-2"-méthyl-propyloxy}-2-hydroxy]-phényl}-6-(4-méthoxyphényl)-1,3,5-triazine.
- la 2,4-bis{[4-(3-(2-propyloxy)-2-hydroxy-propyloxy]-2-hydroxy]-phényl}-6-[(4-éthylcarboxyl)-phénylamino]-1,3,5-triazine ;
- la 2,4-bis{[4-(2-éthylhexyloxy)-2-hydroxy]-phényl}-6-(1-méthylpyrrol-2-yl)-1,3,5-triazine.

Les composés dérivés de bis-résorcinyl triazine plus particulièrement préférés selon l'invention sont choisis dans le groupe constitué par :
- la 2,4-bis{[4-(2-éthylhexyloxy)-2-hydroxy]-phényl}-6-(4-méthoxy-phényl)-1,3,5-triazine ;
- la 2,4-bis{[4-tris(triméthylsiloxy-silylpropyloxy)-2-hydroxy]-phenyl}-6-(4-méthoxyphényl)-1,3,5-triazine ;
- la 2,4-bis{[4-(1',1',1',3',5',5',5'-heptaméthyltrisiloxy-2"-méthyl-propyloxy}-2-hydroxy]-phényl}-6-(4-méthoxyphényl)-1,3,5-triazine.

Le ou les filtres solaires du type dérivés de bis-résorcinyl triazine peuvent être présents dans les compositions selon l'invention à une concentration comprise entre 0,1 et 15%, de préférence entre 0,2 et 10%, en poids par rapport au poids total de la composition.

Parmi les composés comportant au moins deux groupes benzoazolyle conformes à l'invention, on utilisera de préférence ceux répondant à la formule générale (II) suivante : 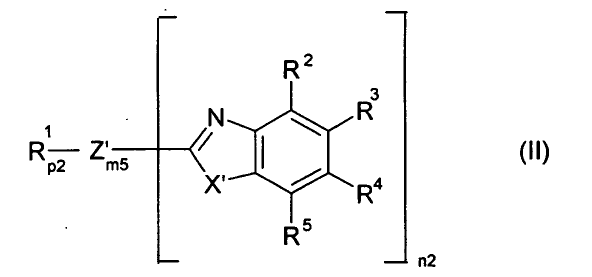 dans laquelle :
- Z' représente un reste organique de valence (p₂ + n₂) comportant une ou plusieurs doubles liaisons placées de telle sorte que qu'elle complète le système de doubles liaisons d'au moins deux groupes benzoazolyle tels que définis à l'intérieur des crochets pour former un en ensemble totalement conjugué ;
- X' désigne S, O ou NR⁶
- R¹ désigne hydrogène, alkyle en C₁-C₁₈, alcoxy en C₁-C₄, un aryle en C₅-C₁₅, un acyloxy en C₂-C₁₈, SO₃Y' ou COOY' ;
- les radicaux R², R³, R⁴ et R⁵, identiques ou différents, désignent un groupe nitro ou ont les mêmes significations que R¹ ;
- R⁶ désigne hydrogène, un alkyle en C₁-C₄ ou un hydroxyalkyle en C₁-C₄ ;
- Y' désigne hydrogène, Li, Na, K, NH₄, 1/2Ca, 1/2Mg, 1/3Al ou un cation résultant de la neutralisation d'un groupe acide libre par une base azotée organique ;
- m₅ est 0 ou 1 ;
- n₂ est un nombre de 2 à 6 ;
- p₂ est un nombre de 1 à 4 ;
- sous réserve que p₂ + n₂ ne dépasse pas la valeur 6.

Les composés de formule (II) conformes à l'invention sont des filtres UV-A hydrosolubles déjà connus dans la demande de brevet EP-A-0669323. Ils sont décrits et préparés selon les synthèses indiquées dans le brevet US 2,463,264 ainsi que la demande de brevet EP-A-0669323 (ces deux documents faisant partie intégrante du contenu de la description).

Parmi les composés de formule (II) de l'invention, on préfère ceux pour lesquels le groupe Z' est choisi dans le groupe constitué par :
(a) un radical hydrocarboné en C₂-C₆ aliphatique linéaire insaturé pouvant être interrompu par un groupe aryle en C₅-C₁₂ ou un hétéroaryle en C₄-C₁₀ comme par exemple :
   -CH=CH-, -CH=CH-CH=CH- ou bien 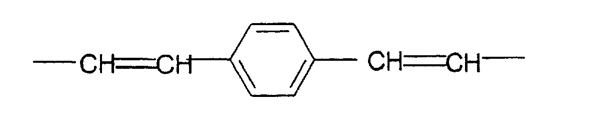
(b) un groupe aryle en C₅-C₁₅ pouvant être interrompu par un radical hydrocarboné en C₂-C₆ aliphatique linéaire insaturé comme par exemple les groupes suivants : 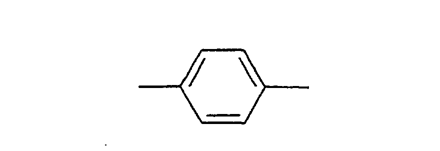 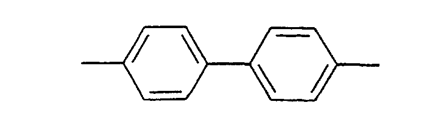 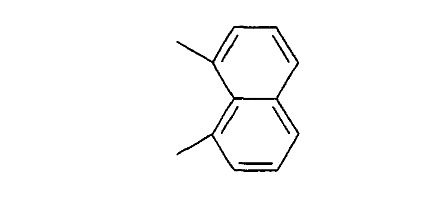 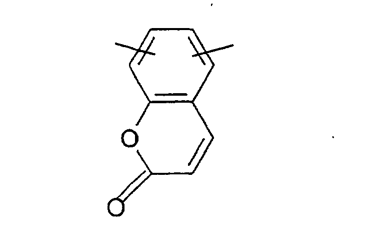 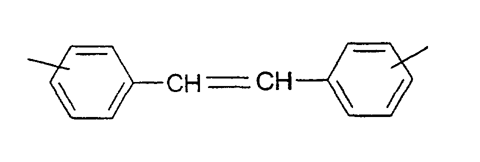
(c) un reste hétéroaryle en C₃-C₁₀ comme par exemple les groupes suivants : 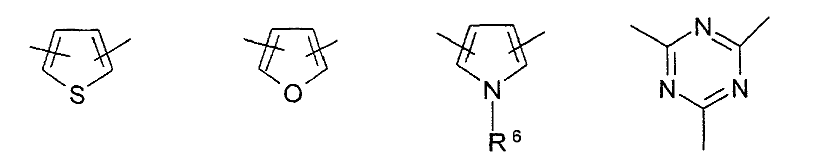
où R⁶ a la même signification indiquée ci-dessus ; lesdits radicaux Z' tels que définis dans les paragraphes (a), (b) et (c) pouvant être substitués par des radicaux alkyle en C₁-C₆, alcoxy en C₁-C₆, phénoxy, hydroxy, méthylènedioxy ou amino éventuellement substitués par un ou 2 radicaux alkyle en C₁-C₅.

A titre d'exemples de composés de formule (II) utilisables, on peut citer ceux de structure suivante ainsi que leurs sels :

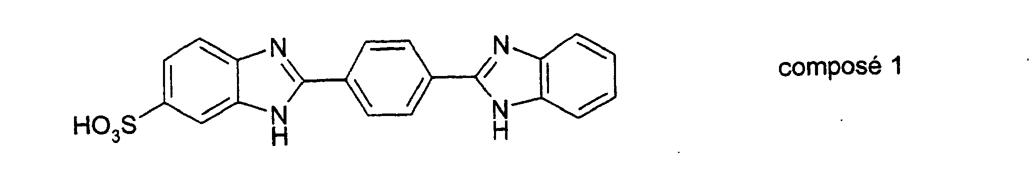

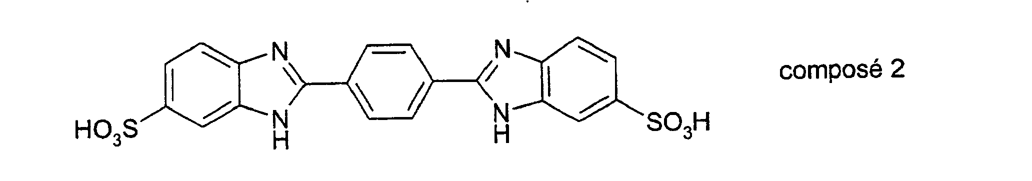

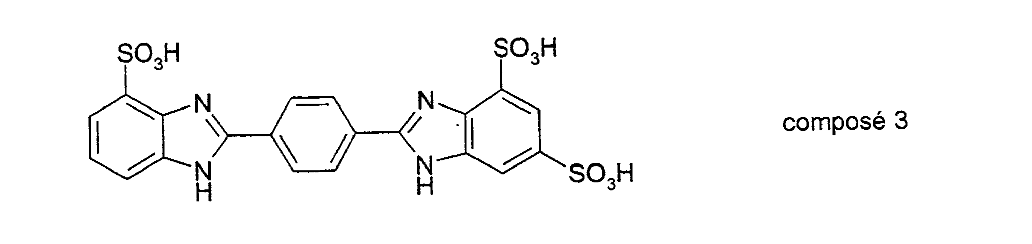

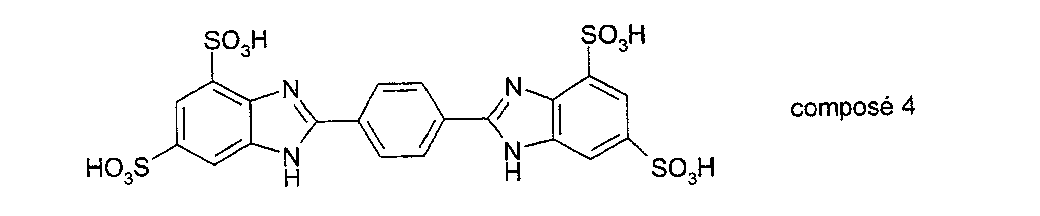

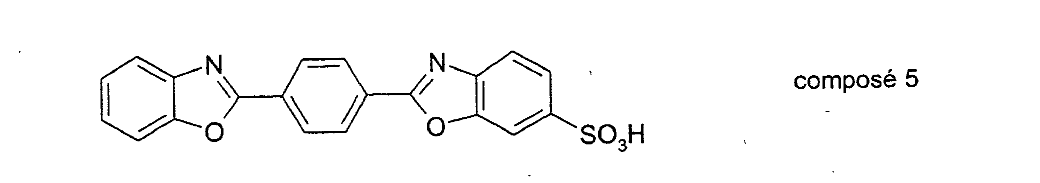

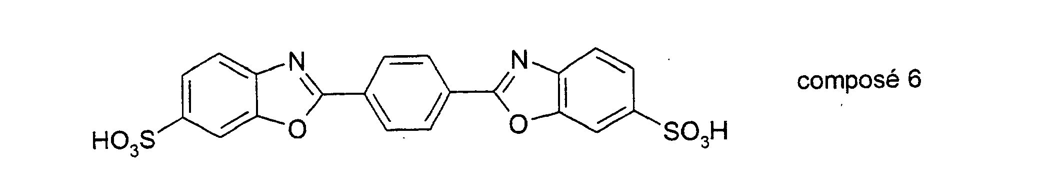

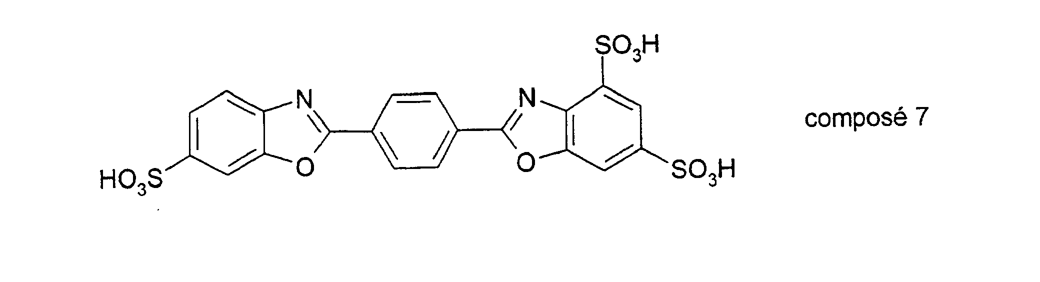

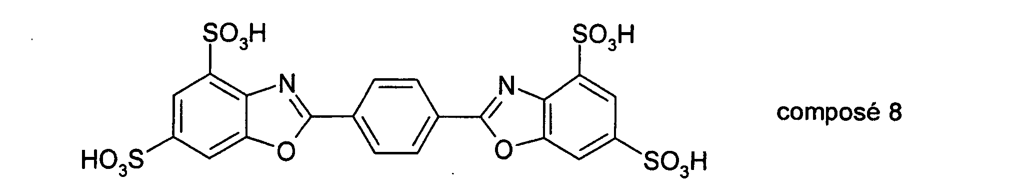

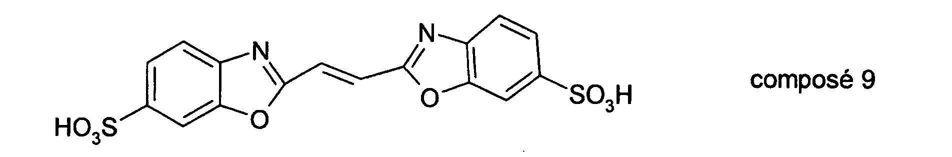

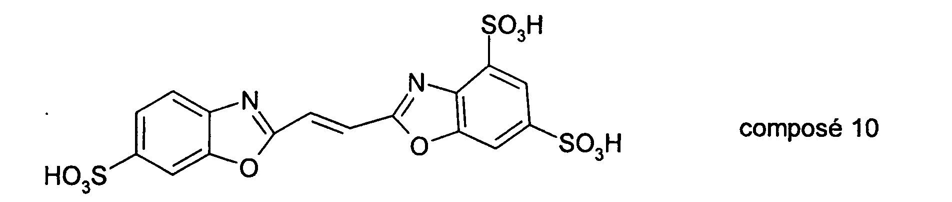

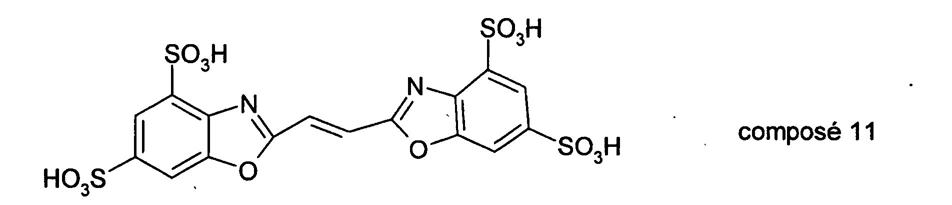

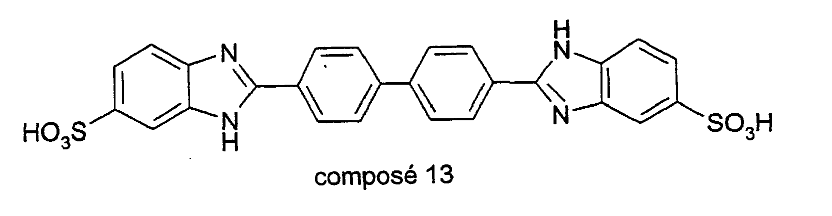

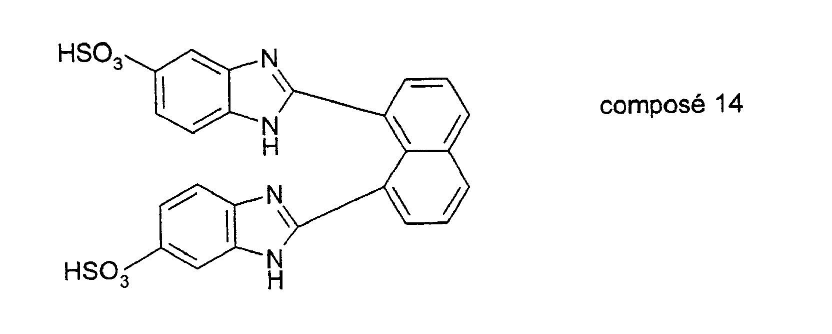

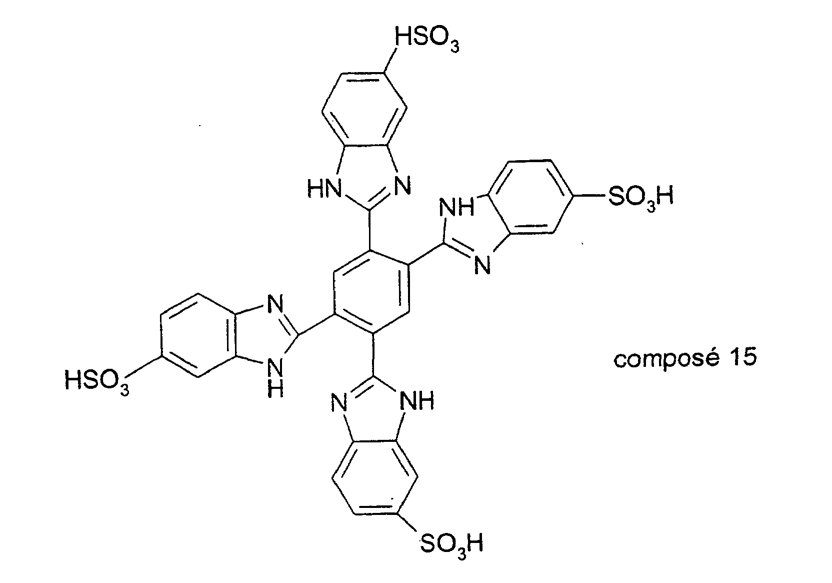

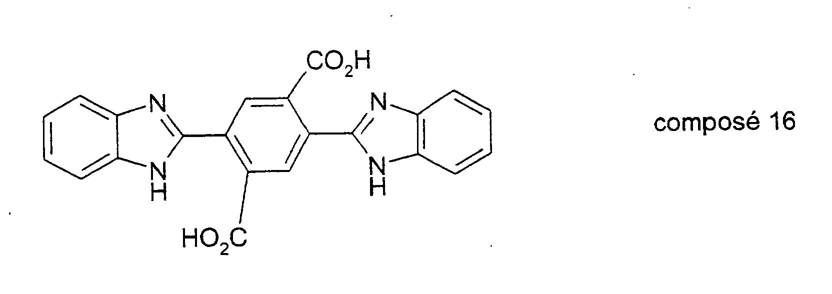

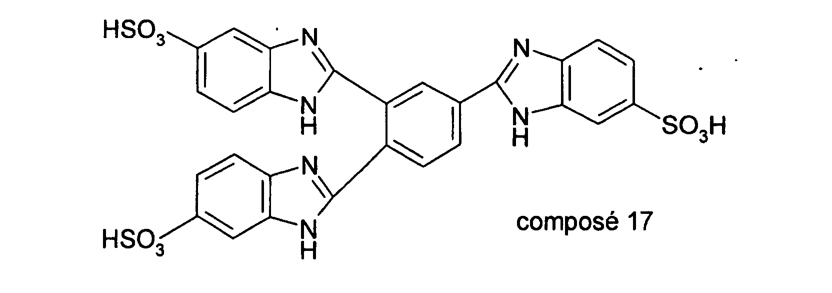

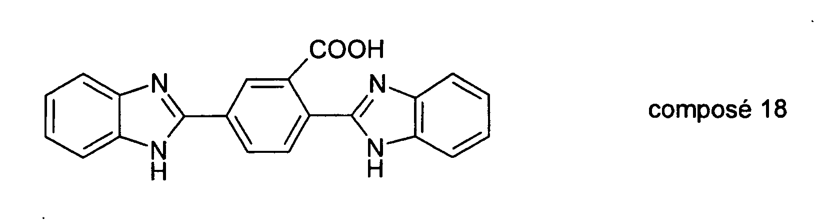

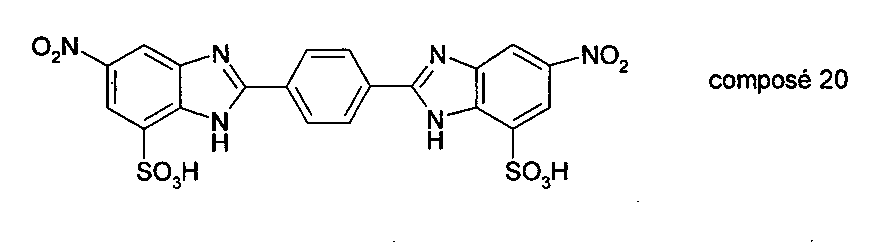

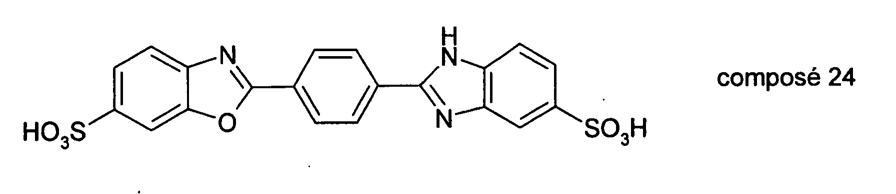

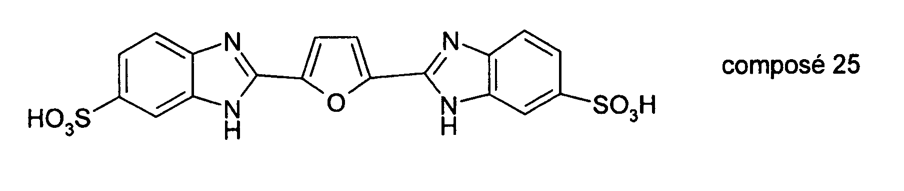

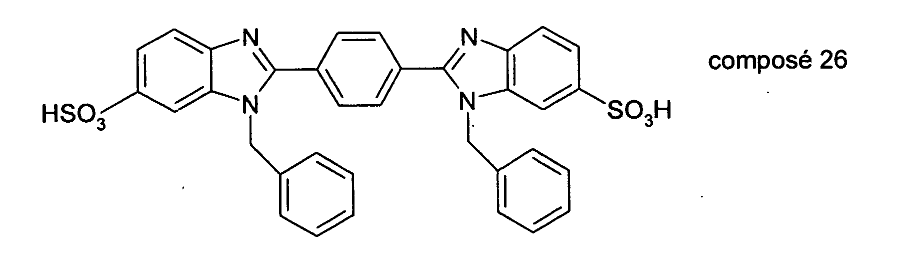

Parmi tous ces composés on préférera tout particulièrement l'acide 1,4-bis-benzimidazolyl-phènylèn-3,3',5,5'-tétrasulfonique (composé 4) ainsi que ses sels de stucture suivante :

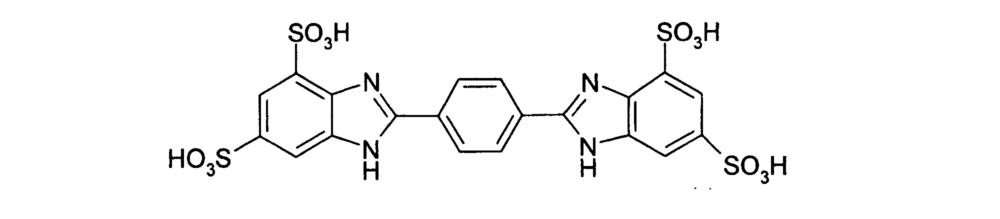

Comme autres exemples de composés comportant au moins deux groupes benzoazolyle, on peut citer également les composés suivants ainsi que leurs sels:

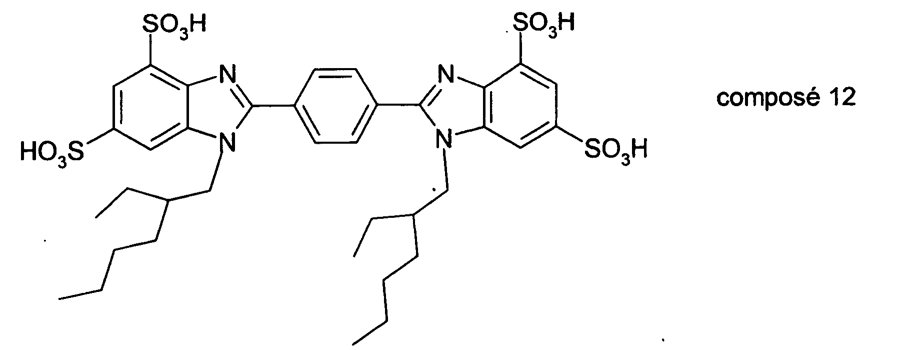

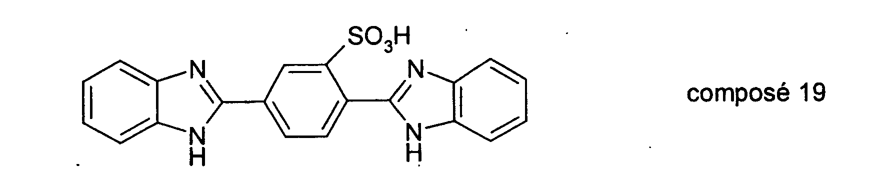

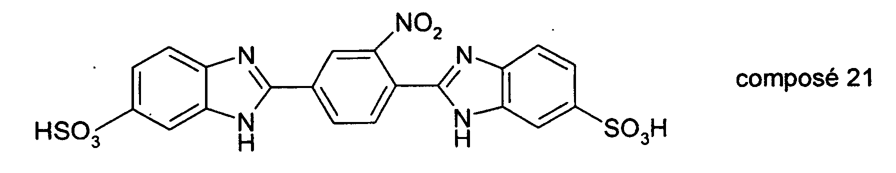

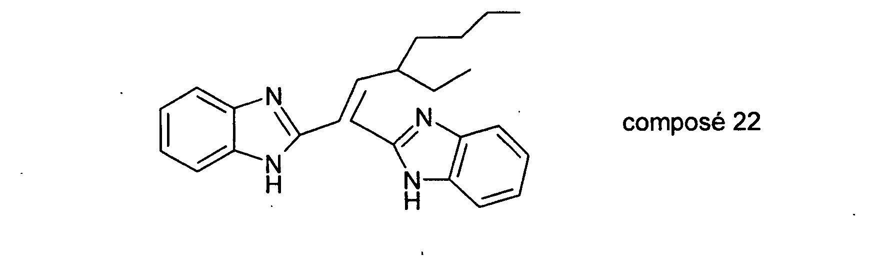

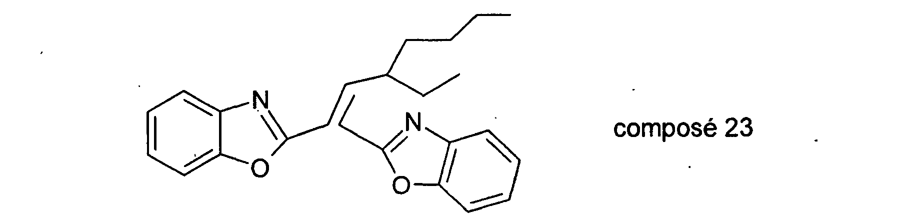

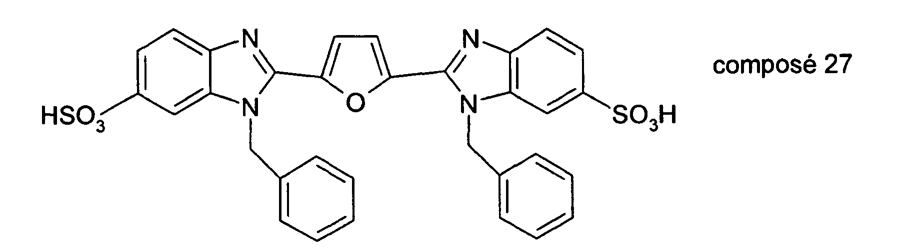

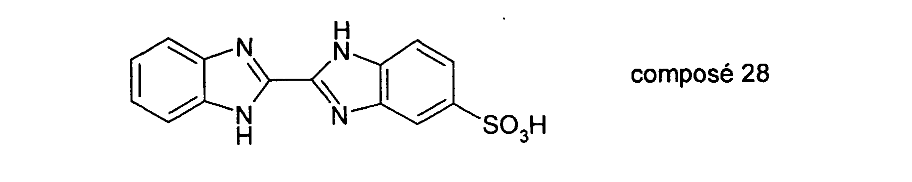

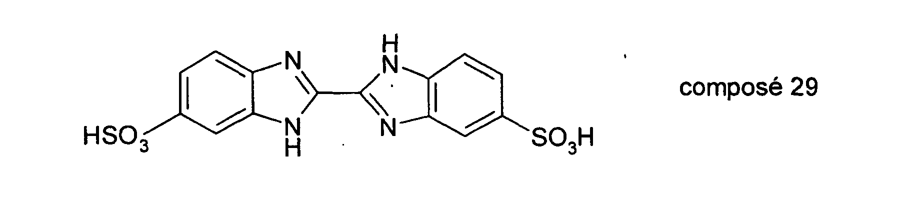

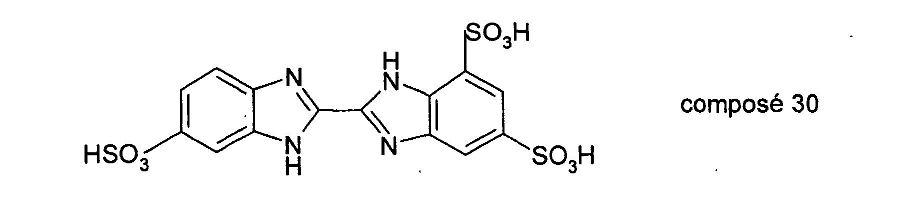

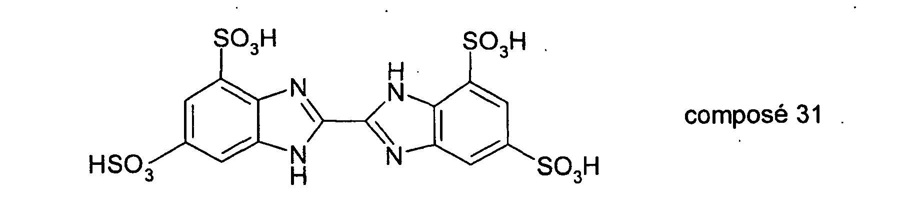

Comme exemples de composés comportant au moins un groupe benzodiazolyle utilisables selon l'invention, on peut mentionner en particulier les composés suivants ainsi que leurs sels :

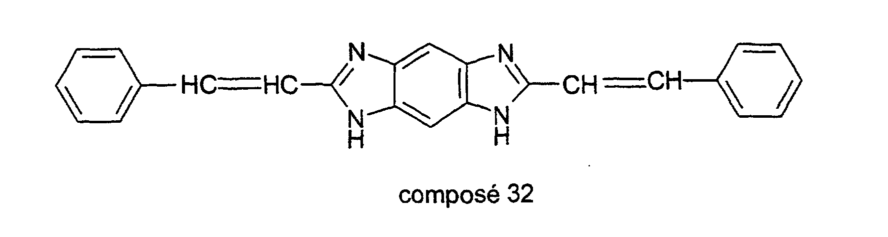

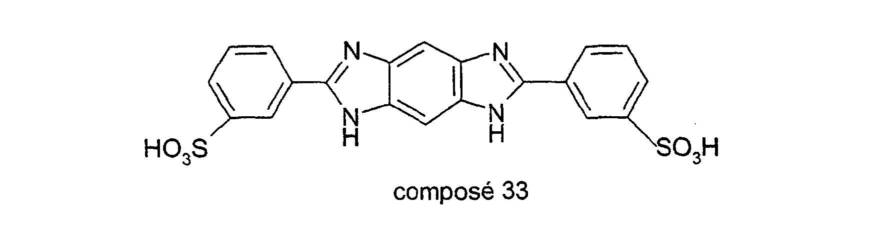

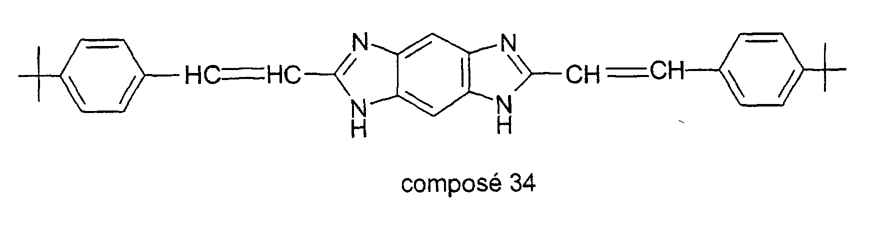

Le ou les composés à groupements benzoazolyle ou benzodiazolyle conformes à l'invention peuvent être présents dans les compositions selon l'invention à une concentration comprise entre 0,1 et 15%, de préférence entre 0,2 et 10%, en poids par rapport au poids total de la composition.

Comme indiqué précédemment, selon une caractéristique essentielle de la présente invention, il convient que les deux types de filtres solaires soient tous présents dans la composition finale dans une proportion respective telle qu'un effet de synergie au niveau de l'indice de protection conféré par l'association résultante, soit obtenu de manière notable, substantielle et significative.

En outre, et d'une manière générale, on notera que les concentrations et rapports en dérivés de bis-résorcinyl triazine et composés à groupements benzoazolyle ou benzodiazolyle tels que définis précédemment sont choisis de manière telle que l'indice de protection solaire de la composition finale soit de préférence d'au moins 2.

Selon un mode préféré de réalisation de la présente invention, le support cosmétiquement acceptable dans lequel se trouvent contenus les différents types de filtres est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques ; les dérivés salicyliques, les dérivés du camphre ; les dérivés de triazine autres que ceux définis précédemment tels que ceux décrits dans les demandes de brevet EP863145, EP517104, EP570838 et EP796851 ; les dérivés de la benzophénone ; les dérivés du dibenzoylméthane ; les dérivés de β,β'-diphénylacrylate ; les dérivés de benzimidazole autres que ceux décrits précédemment ; les dérivés de l'acide p-aminobenzoïque ; les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide urocanique,
l'acide benzène 1,4-di(3-méthylidène-10-camphosulfonique) et ses sels,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
la 2,4,6-tris-[p-(2'-éthylhexyl-1'-oxycarbonyl)anilino]-1,3,5-triazine,
2-[p-(tertiobutylamido)anilino]-4,6-bis-[(p-(2'-éthylhexyl-1'-oxycarbonyl)anilino]-1,3,5-triazine,
le polymère de N-[(2 et 4)-[(2-oxoborn-3-ylidèn)méthyl] benzyl]-acrylamide,
Le drométrizole trisiloxane (nom INCI),
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier les indices de protection solaire, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLES

| **COMPOSITION** | **EX 1** |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en C₁₂/C₁₅ (WITCONOL TN -WITCO) | 15g |
| 2,4-bis-{[4-(2-ethyl-hexyloxy)-2-hydroxy]-phenyl}-6-(4-methoxyphenyl)-1,3,5-triazine | 2g |
| Glycérine | 15g |
| Acide 1,4-bis-benzimidazolyl-phènylène-3,3',5,5'-tétrasulfonique | 2g |
| Triéthanolamine | qs pH 7 |
| Conservateurs | qs |
| Eau déminéralisée qsp | 100 g |

| **COMPOSITION** | **EX 2** |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en C₁₂/C₁₅ (WITCONOL TN -WITCO) | 20g |
| Triéthanolamine | 0.5g |
| 2,4-Bis-{[4-(tris(trimethylsiloxy)silylpropyloxy)-2-hydroxy]-phenyl}-6-(4-methoxyphenyl)-1,3,5-triazine | 2.5g |
| Glycérine | 5g |
| Phosphate d'alcool hexadécylique,sel de potassium (AMPHISOL K - HOFFMAN LAROCHE) | 1g |
| Acide polyacrylique (SYNTHALEN K - 3V) | 0.3g |
| Hydroxypropyl méthyl cellulose (METHOCEL F4M -DOW CHEMICAL) | 0.1g |
| Acide 1,4-bis-benzimidazolyl-phènylène-3,3',5,5'-tétrasulfonique | 1.5g |
| Triéthanolamine | qs pH 7 |
| Conservateurs | qs |
| Eau démineralisée qsp | 100 g |

| **COMPOSITION** | **EX 3** |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en C₁₂/C₁₅ (WITCONOL TN -WITCO) | 15g |
| 2,4-Bis-{[4-(1',1',1',3',5',5',5'-Heptamethyltrisiloxy-2''-methylpropyloxy)-2-hydroxy]-phenyl}-6-(4-methoxyphenyl)-1,3,5-triazine | 2g |
| Glycérine | 15g |
| Acide 1,4-bis-benzimidazolyl-phènylène-3,3',5,5'-tétrasulfonique | 1.5g |
| Triethanolamine | qs pH 7 |
| Conservateurs | qs |
| Eau déminéralisée qsp | 100 g |

## Revendications

1. Composition cosmétique à usage topique, pour la photoprotection de la peau et/ou des cheveux, **caractérisée par le fait qu'**elle comprend, dans un support cosmétiquement acceptable:
(a) à titre de premier filtre, au moins un dérivé de bis-résorcinyl triazine de formule (I) suivante : 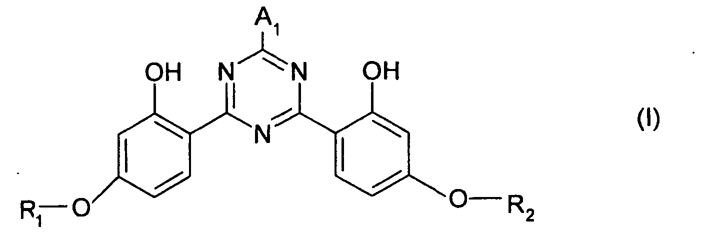 dans laquelle :
(i) les radicaux R₁ et R₂, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ;
(ii) les radicaux R₁ et R₂, identiques ou différents, peuvent aussi désigner un reste de formule (1) suivante : 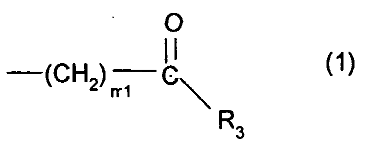 dans laquelle:
- m₁ est un nombre de 1 à 3 ;
- R₃ désigne un groupe hydroxy ; un radical alkyle en C₁-C₅ non substitué ou substitué par un ou plusieurs groupes hydroxy ; un radical alcoxy en C₁-C₅ ; un groupe amino ; un radical mono- ou dialkyl(C₁-C₅)amino ; un cation métallique M ; un reste choisi parmi l'une des formules (2) à (7) suivantes : 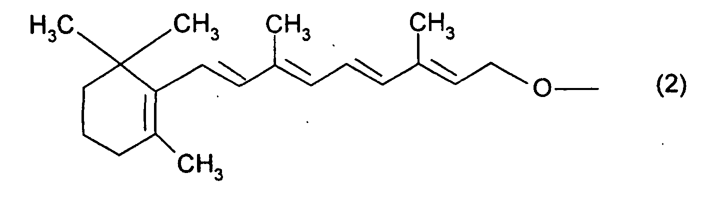 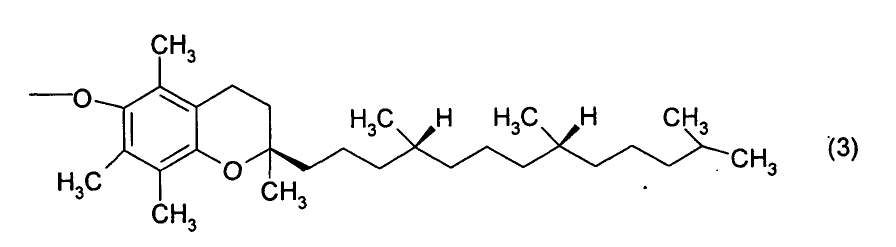 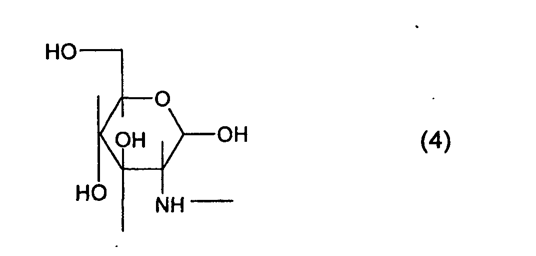 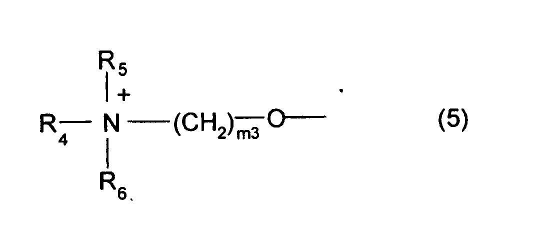 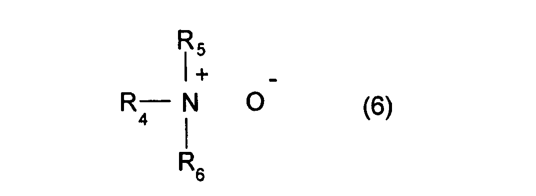 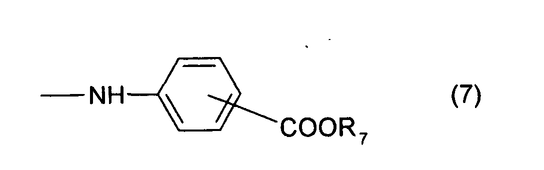
dans lesquelles
- les radicaux R₄, R₅ et R₆, identiques ou différents, désignent un radical alkyle en C₁-C₁₄ non substitué ou substitué par un ou plusieurs groupes hydroxy ;
- m₃ est un nombre de 2 à 14 ;
- R₇ représente un atome d'hydrogène, un cation métallique M, un radical alkyle en C₁-C₅ ou un reste de formule -(CH₂)ₘ₂-OT₁ où m₂ est un nombre de 1 à 4 et T₁ a la même signification indiquée ci-dessus ;
(iii) les radicaux R₁ et R₂, identiques ou différents, peuvent encore désigner un reste de formule (8) suivante : 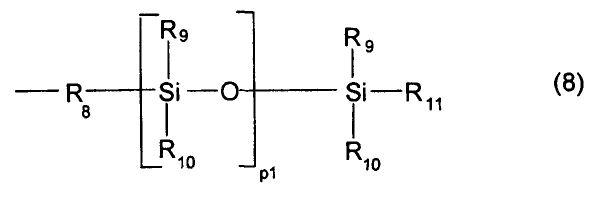 dans laquelle :
- R₈ désigne une liaison covalente ; un radical alkyle linéaire ou ramifié en C₁-C₄ ou bien un reste de formule -Cₘ₄H₂ₘ₄- ou -Cₘ₄H₂ₘ₄-O- où m₄ est un nombre de 1 à 4 ;
- p₁ est un nombre de 0 à 5 ;
- les radicaux R₉, R₁₀ et R₁₁, identiques ou différents, désignent un radical alkyle en C₁-C₁₈ ; un alcoxy en C₁-C₁₈ ou un reste de formule : 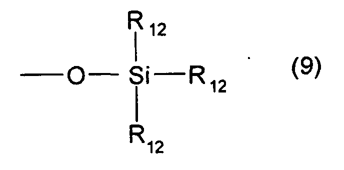
où R₁₂ est un radical alkyle en C₁-C₅ ;
- A₁ désigne un reste répondant à l'une des formules suivantes : 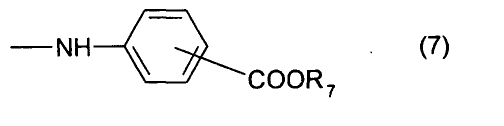 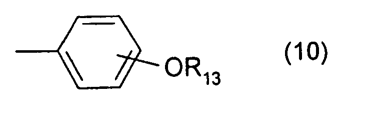 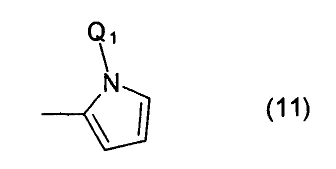 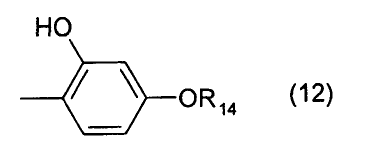
dans lesquelles :
- R₇ a les mêmes significations indiquées ci-dessus ;
- R₁₃ désigne un atome d'hydrogène, un radical alkyle en C₁-C₁₀, un radical de formule :
-(CH₂CHR₁₆-O)ₙ₁R₇ où n₁ est un nombre de 1 à 16, R₁₆ est hydrogène ou méthyle ou bien un reste de structure -CH₂-CH-(OH)-CH₂OT₁ avec T₁ ayant la même signification indiquée ci-dessus.
- Q₁ est un radical alkyle en C₁-C₁₈ ;
- R₁₄ est un radical répondant à la formule (1) : 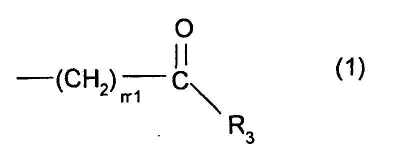
(b) à titre de deuxième filtre, au moins un composé comportant par molécule au moins deux groupes benzoazolyle de formule (II) suivante : 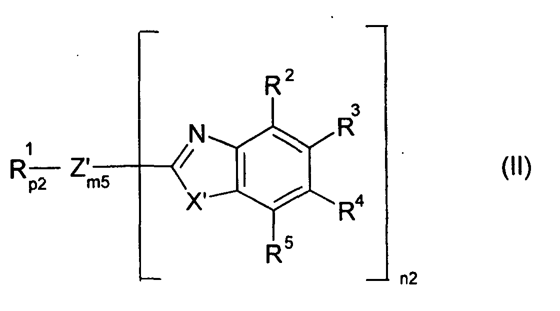 dans laquelle :
- Z' choisi dans le groupe constitué par :
(i) un radical hydrocarboné en C₂-C₆ aliphatique linéaire oléfine pouvant être interrompu par un groupe aryle en C₅-C₁₂ ou un hétéroaryle en C₄-C₁₀
(ii) un groupe aryle en C₅-C₁₅ pouvant être interrompu par un radical hydrocarboné en C₂-C₆ aliphatique linéaire oléfine ;
(iii) un groupe hétéroaryle en C₃-C₁₀ ; ledit radical Z' pouvant être substitué par des radicaux alkyle en C₁-C₆, alcoxy en C₁-C₆, phénoxy, hydroxy, méthylènedioxy ou amino, éventuellement substitués par un ou 2 radicaux alkyle en C₁-C₅ ;
- X' désigne S, O ou NR⁶
- R¹ désigne hydrogène, alkyle en C₁-C₁₈, alcoxy en C₁-C₄, un aryle en C₅-C₁₅, un acyloxy en C₂-C₁₈, SO₃Y' ou COOY' ;
- les radicaux R², R³, R⁴ et R⁵, identiques ou différents, désignent un groupe nitro ou ont les mêmes significations que R₁ ;
- R⁶ désigne hydrogène, un alkyle en C₁-C₄ ou un hydroxyalkyle en C₁-C₄ ;
- Y' désigne hydrogène, Li, Na, K, NH₄, 1/2Ca, 1/2Mg, 1/3Al ou un cation résultant de la neutralisation d'un groupe acide libre par une base azotée organique ;
- m₅ est 0 ou 1 ;
- n₂ est un nombre de 2 à 6 ;
- p₂ est un nombre de 1 à 4 ;
- sous réserve que p₂ + n₂ ne dépasse pas la valeur 6;
lesdits premier et deuxième filtres étant présents dans une proportion produisant une activité synergique au niveau des indices de protection solaire conférés.

2. Composition cosmétique à usage topique, pour la photoprotection de la peau et/ou des cheveux, **caractérisée par le fait qu'**elle comprend, dans un support cosmétiquement acceptable :
(a) à titre de premier filtre, au moins un dérivé de bis-résorcinyl triazine de formule (I) telle que définie dans la revendication 1 ;
(b) à titre de deuxième filtre, au moins un composé choisi parmi les composés suivants ou l'un de leurs sels : 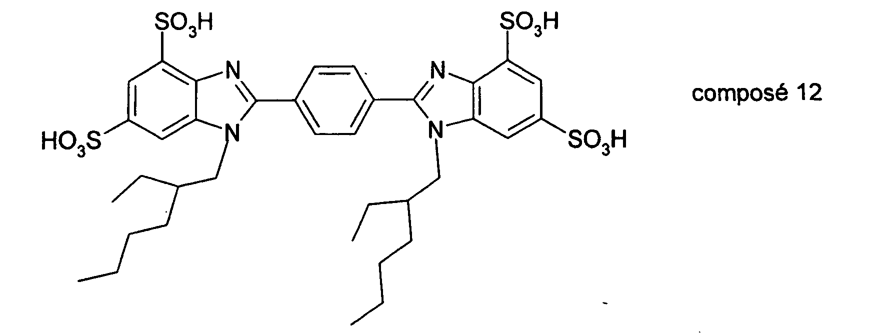 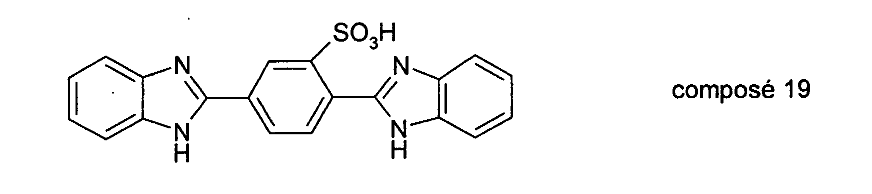 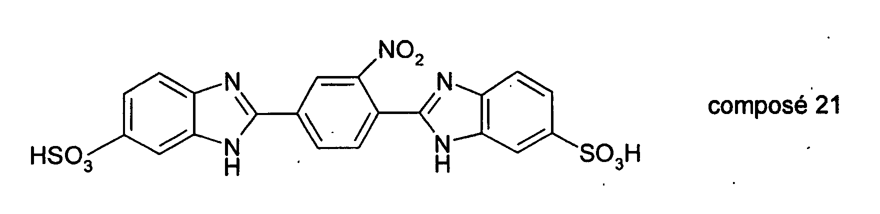 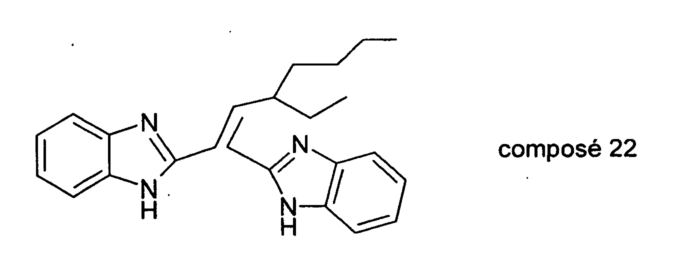 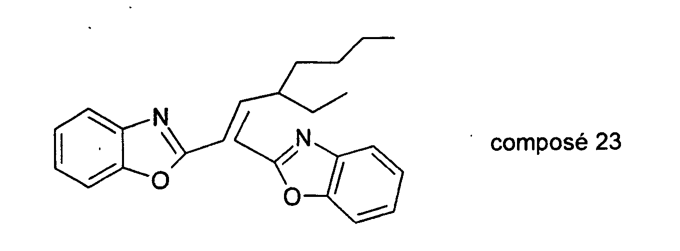 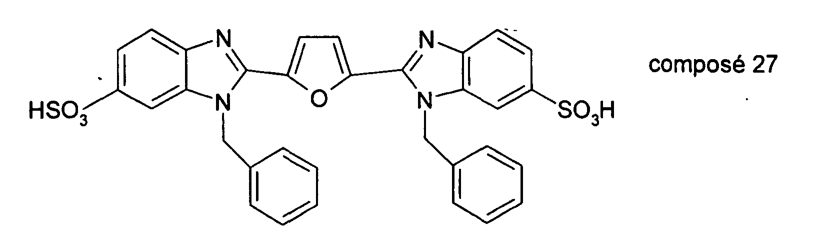 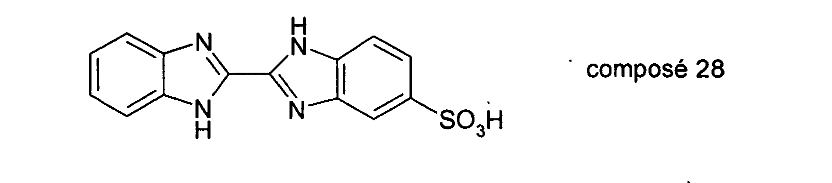 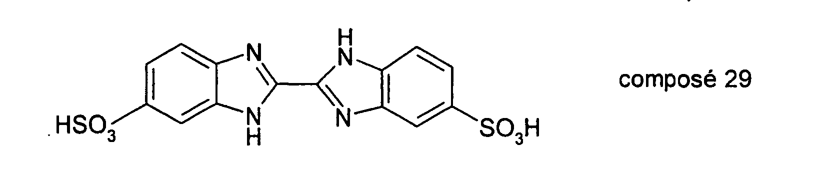 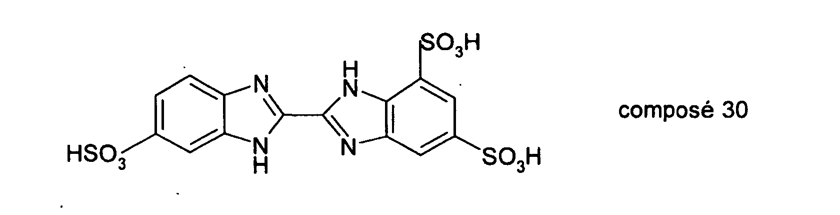 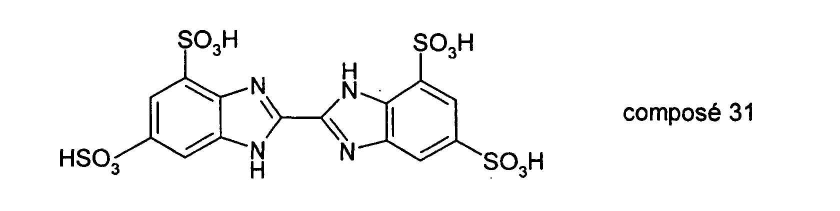 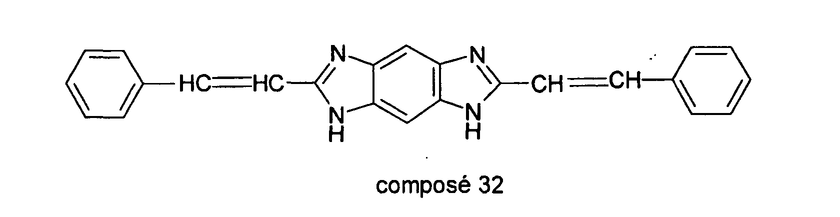 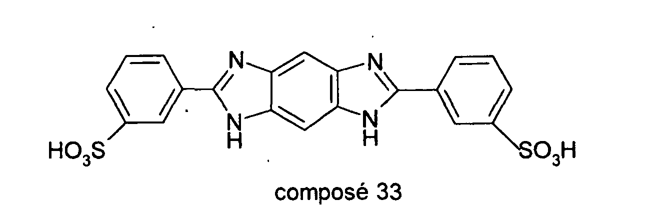 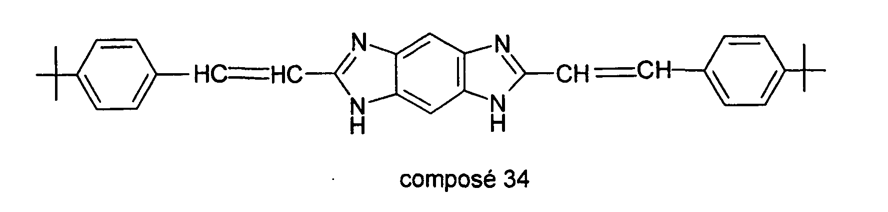
; lesdits premier et deuxième filtres étant présents dans une proportion produisant une activité synergique au niveau des indices de protection solaire conférés.

3. Composition selon la revendication 1 ou 2, **caractérisée par le fait que** le composé de formule (I) est choisi parmi :
(1) ceux pour lesquels le radical A₁ désigne para-méthoxyphényle ou para-éthoxyphényle et les radicaux R₁ et R₂, identiques ou différents, désignent un radical de structure : 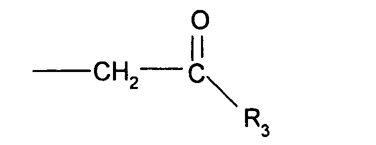 dans laquelle R₃ est choisi parmi :
- tert.butyloxy ;
- OH ;
- OM où M est un cation alcalin, alcalinoterreux ou choisi parmi le cuivre, le magnésium ou le zinc ;
- un groupe de structure : 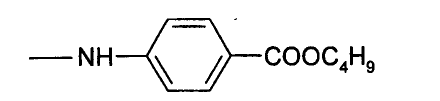
- un groupe de structure O⁻N⁺(CH₂CH₂OH)₃ ;
- un groupe de structure : 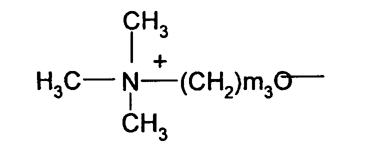 avec m₃ variant de 2 à 14;
- un groupe de structure : 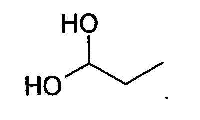
- un groupe de structure : 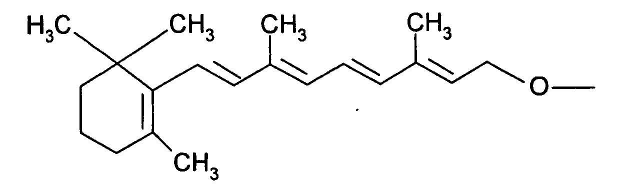
(2) le composé bis-résorcinyl triazine répondant à la formule (I) dans laquelle le radical A₁ désigne para-hydroxyphényle et les radicaux R₁ et R₂ désignent simultanément un groupe de structure :

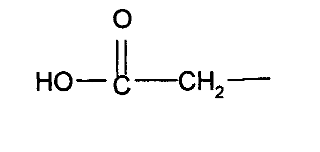

4. Composition selon la revendication 3, **caractérisée par le fait que** le composé de formule (I) est choisi dans le groupe constitué par :
- la 2,4-bis{[4-(2-éthylhexyloxy)-2-hydroxy]-phényl}-6-(4-méthoxy-phenyl)-1,3,5-triazine ;
- la 2,4-bis{[4-(3-(2-propyloxy)-2-hydroxy-propyloxy)-2-hydroxy]-phényl}-6-(4-méthoxyphényl)-1,3,5-triazine ;
- la 2,4-bis{[4-(2-éthyl-hexyloxy)-2-hydroxy]-phényl}-6-[4-(2-méthoxyéthylcarboxyl)-phénylamino]-1,3,5-triazine ;
- la 2,4-bis{[4-tris(triméthylsiloxy-silylpropyloxy)-2-hydroxy]-phényl}-6-(4-méthoxyphényl)-1,3,5-triazine ;
- la 2,4-bis{[4-(2"-méthylpropenyloxy)-2-hydroxy]-phényl}-6-(4-méthoxyphényl)-1,3,5-triazine ;
- la 2,4-bis{[4-(1',1',1',3',5',5',5'-heptaméthyltrisiloxy-2"-méthyl-propyloxy}-2-hydroxy]-phényl}-6-(4-méthoxyphényl)-1,3,5-triazine.
- la 2,4-bis{[4-(3-(2-propyloxy)-2-hydroxy-propyloxy]-2-hydroxy]-phényl}-6-[(4-éthylcarboxyl)-phénylamino]-1,3,5-triazine ;
- la 2,4-bis{[4-(2-éthyl-hexyloxy)-2-hydroxy]-phényl}-6-(1-méthylpyrrol-2-yl)-1,3,5-triazine.

5. Composition selon la revendication 4, **caractérisée par le fait que** le composé de formule (I) est choisi dans le groupe constitué par :
- la 2,4-bis{[4-(2-éthyl-hexyloxy)-2-hydroxy]-phényl}-6-(4-méthoxy-phenyl)-1,3,5-triazine ;
- la 2,4-bis{[4-tris(triméthylsiloxy-silylpropyloxy)-2-hydroxy]-phenyl}-6-(4-méthoxyphenyl)-1,3,5-triazine ;
- la 2,4-bis{[4-(1',1',1',3',5',5',5'-heptaméthyltrisiloxy-2"-méthyl-propyloxy}-2-hydroxy]-phenyl}-6-(4-méthoxyphenyl)-1,3,5-triazine.

6. Composition selon l'une quelconque des revendications précédentes, **caractérisée par le fait que** la concentration en dérivé de bis-résorcyl triazine est comprise entre 0,1 et 15 % en poids par rapport au poids total de la composition.

7. Composition selon la revendication 6, **caractérisée par le fait que** ladite concentration est comprise entre 0,2 et 10 % en poids par rapport au poids total de la composition.

8. Composition selon l'une quelconque des revendications 1 et 3 à 7, où le radical Z' est choisi dans le groupe constitué par les groupes suivants :
-CH=CH-
-CH=CH-CH=CH- 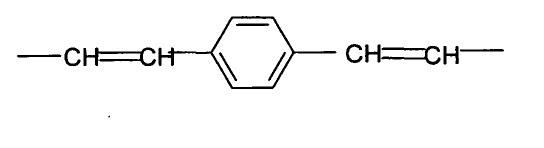 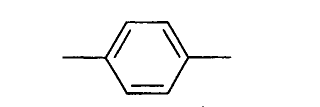 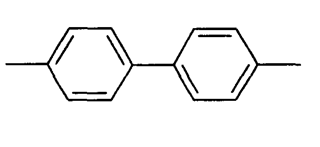 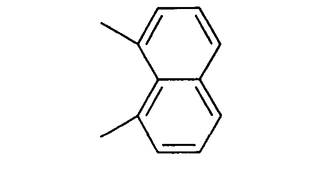 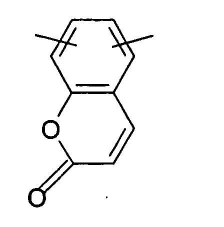 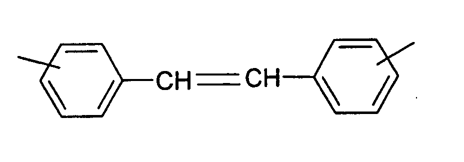 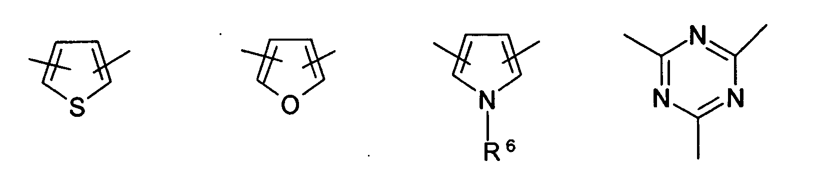
avec R⁶ ayant la même signification indiquée dans la revendication 1.

9. Composition selon l'une quelconque des revendications 1 et 3 à 7, où le composé de formule (II) est choisi parmi les composés suivants ou l'un de leurs sels :

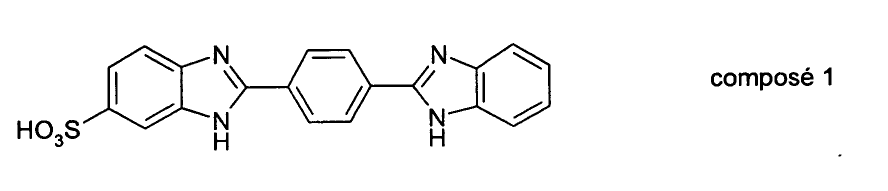

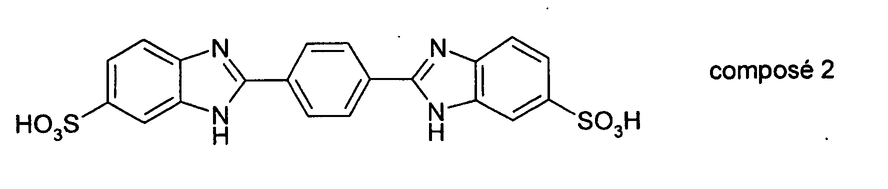

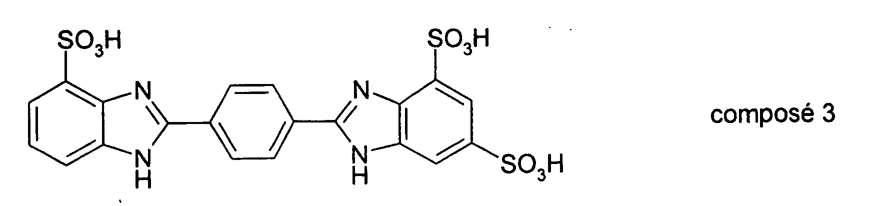

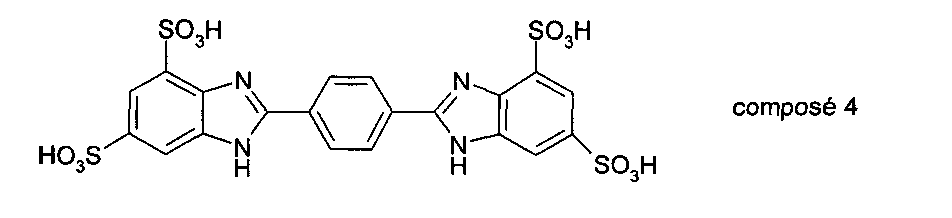

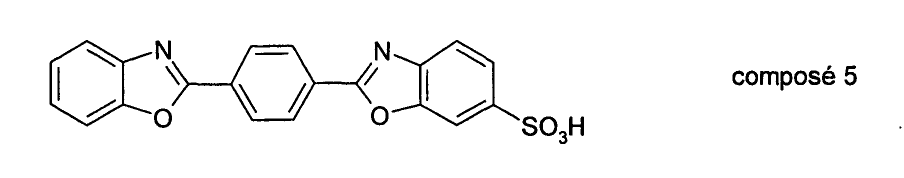

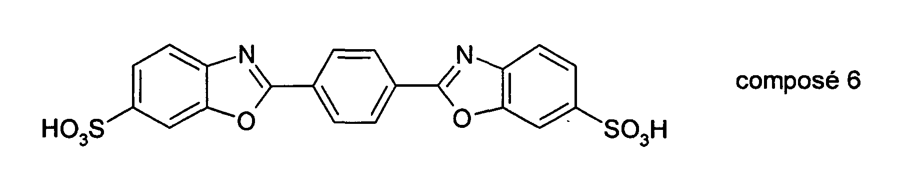

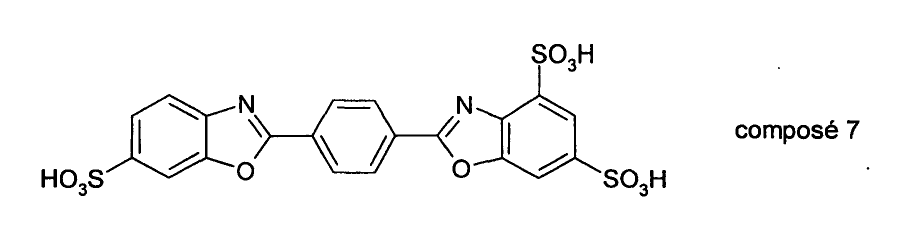

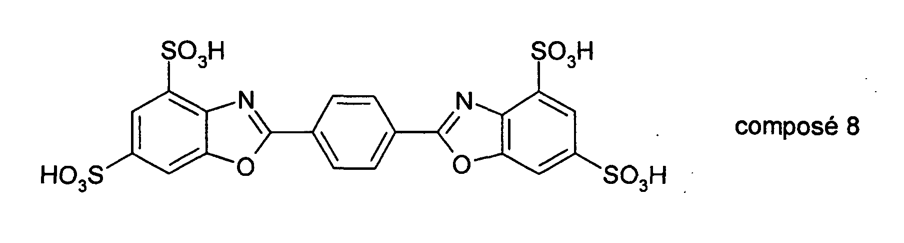

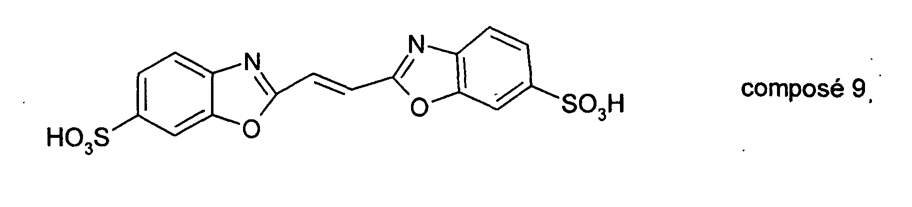

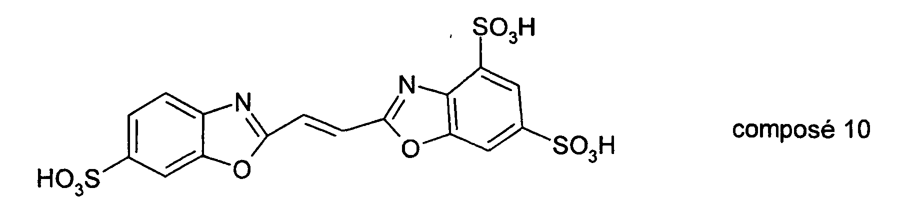

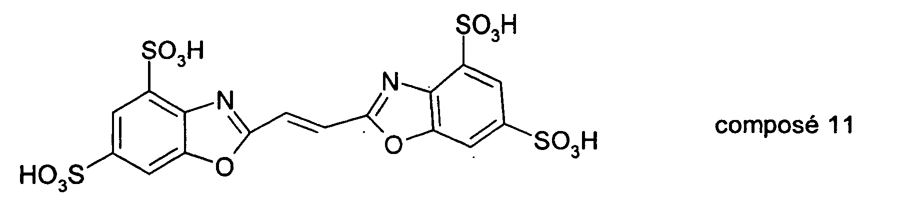

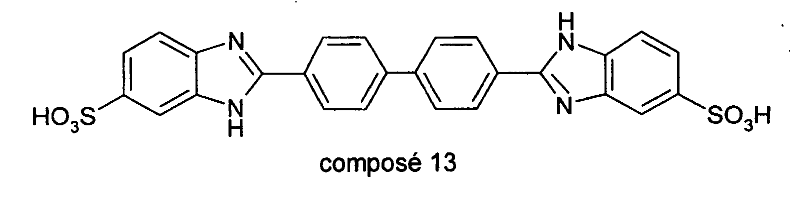

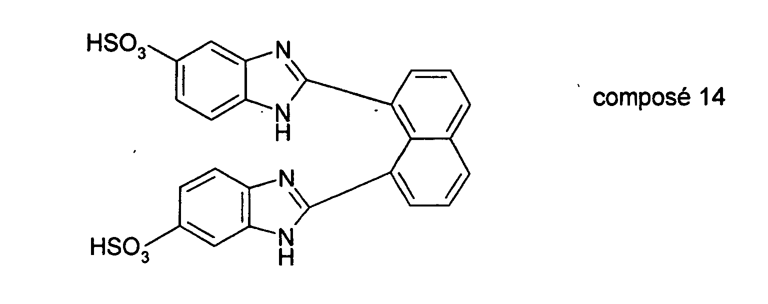

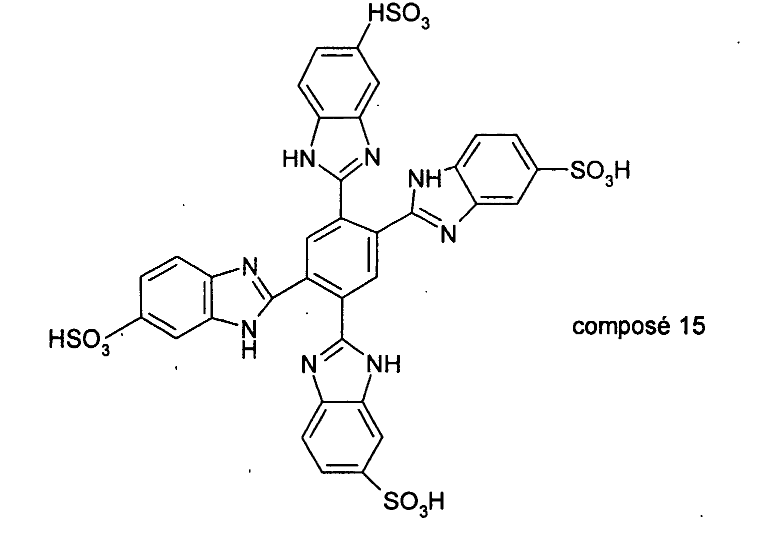

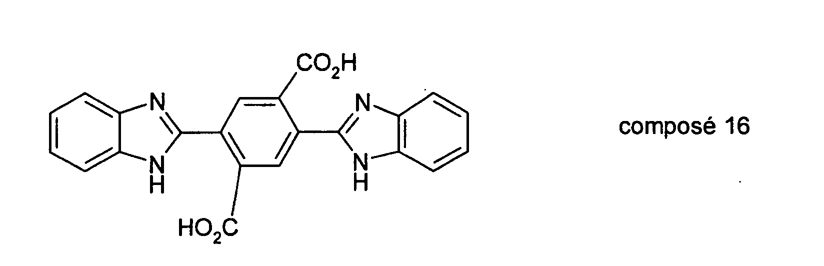

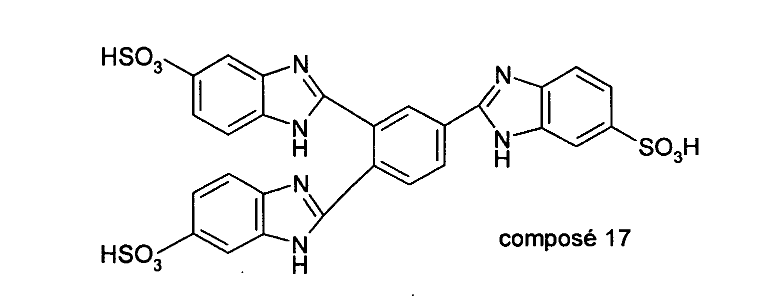

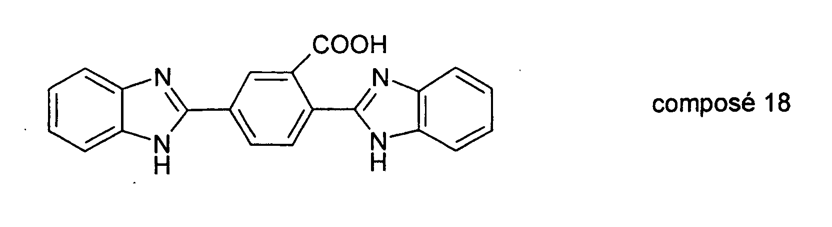

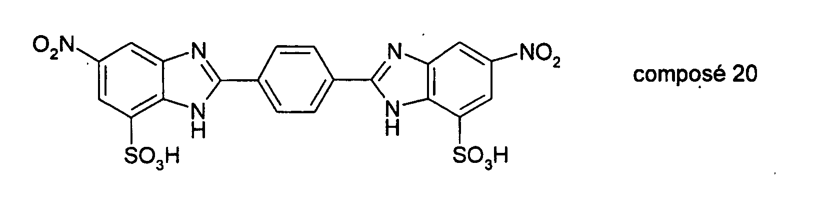

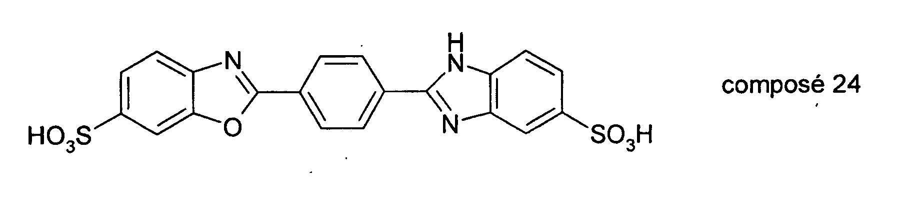

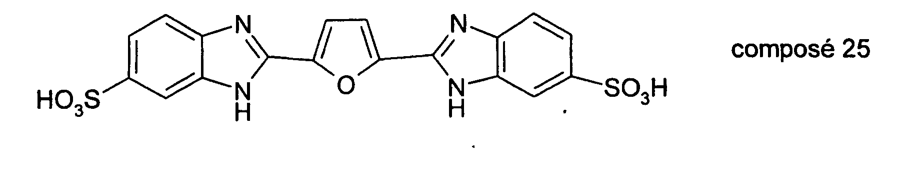

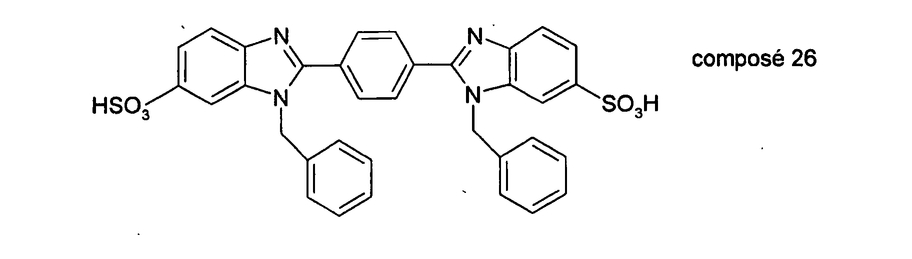

10. Composition selon la revendications 9, où le composé de formule (II) est l'acide phènylène-1,4-bis-benzimidazole-3,3',5,5'-tétrasulfonique ou l'un des ses sels de structure suivante:

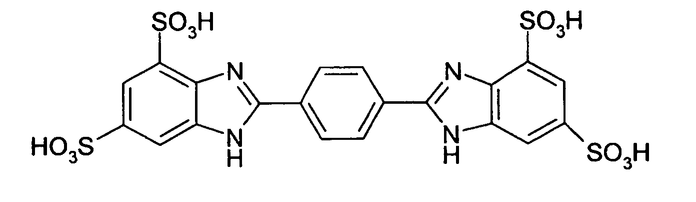

11. Composition selon l'une quelconque des revendications 1 à 10, **caractérisée par le fait que** le ou les composés de formule (II) tels que définis dans l'une queconque des revendications 1 et 3 à 10 ou bien le ou les composés tels que définis dans la revendication 2 sont présents à une concentration comprise entre 0,1 et 15 % en poids par rapport au poids total de la composition.

12. Composition selon la revendication 11, **caractérisée par le fait que** ladite concentration est comprise entre 0,2 et 10 % en poids par rapport au poids total de la composition.

13. Composition selon l'une quelconque des revendications 1 à 12, **caractérisée par le fait que** ledit support cosmétiquement acceptable se présente sous la forme d'une émulsion de type huile-dans-eau.

14. Composition selon l'une quelconque des revendications 1 à 13, **caractérisée par le fait qu'**elle comprend en outre un ou plusieurs filtres organiques complémentaires actifs dans l'UV-A et/ou UV-B, hydrophiles ou lipophiles, différents desdits premier et deuxième filtres.

15. Composition selon la revendication 14, **caractérisée par le fait que** lesdits filtres organiques complémentaires sont choisis parmi les dérivés cinnamiques, les dérivés salicyliques, les dérivés du camphre, les dérivés de triazine autres que ceux définis dans les revendications précédentes, les dérivés de benzimidazole autres que ceux définis dans les revendications précédentes, les dérivés de la benzophénone, les dérivés du dibenzoylméthane, les dérivés de β,β'-diphénylacrylate, les dérivés de l'acide p-aminobenzoïque, les polymères filtres et silicones filtres.

16. Composition selon l'une quelconque des revendications 1 à 15, **caractérisée par le fait qu'**elle comprend en outre, à titre d'agents photoprotecteurs UV complémentaires, des pigments ou des nanopigments d'oxydes métalliques, enrobés ou non.

17. Composition selon la revendication 16, **caractérisée par le fait que** lesdits pigments ou nanopigments sont choisis parmi les oxydes de titane, de zinc, de fer, de zirconium, de cérium et leurs mélanges, enrobés ou non.

18. Composition selon l'une quelconque des revendications 1 à 17, **caractérisée par le fait qu'**elle comprend en outre au moins un agent de bronzage et/ou de brunissage artificiel de la peau.

19. Composition selon l'une quelconque des revendications 1 à 18, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

20. Composition selon l'une quelconque des revendications 1 à 19**, caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

21. Composition selon l'une quelconque des revendications 1 à 20 **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

22. Composition selon l'une quelconque des revendications 1 à 20, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

23. Utilisation de la composition définie selon l'une quelconque des revendications 1 à 22 pour la fabrication de compositions cosmétiques pour la protection de la peau ou des cheveux contre le rayonnement ultraviolet, en particulier le rayonnement solaire.

24. Procédé de traitement cosmétique pour protéger la peau et/ou les cheveux contre le rayonnement ultraviolet, **caractérisé en ce qu'**il consiste à appliquer sur ceux-ci une quantité efficace d'une composition telle que définie à l'une quelconque des revendications 1 à 22.

## Patentansprüche

1. Kosmetische Zusammensetzung zur topischen Anwendung für den Lichtschutz der Haut und/oder der Haare, **dadurch gekennzeichnet, dass** sie in einem kosmetisch akzeptablen Träger enthält:
(a) als erstes Filter mindestens ein spezielles Bis-resorcinyltriazinderivat der folgenden Formel (I): 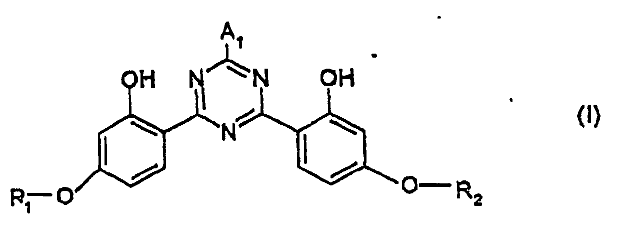 worin:
(i) die Gruppen R₁ und R₂, die gleich oder verschieden sind, eine C₃₋₁₈-Alkylgruppe, eine C₂₋₁₈-Alkenylgruppe oder einen Rest der Formel -CH₂-CH(OH)-CH₂-OT₁ bedeuten, wobei T₁ ein Wasserstoffatom oder eine C₁₋₈-Alkylgruppe bedeutet;
(ii) die Gruppen R₁ und R₂, die gleich oder verschieden sind, auch eine Gruppe der folgenden Formel (1) bedeuten können: 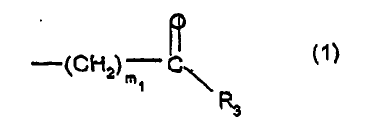 worin bedeuten:
- m₁ eine Zahl von 1 bis 3,
- R₃ eine Hydroxygruppe, eine unsubstituierte oder mit einer oder mehreren Hydroxygruppen substituierte C₁₋₅-Alkylgruppe, eine C₁₋₅-Alkoxygruppe, eine Aminogruppe, eine Mono- oder Dialkyl(C₁₋₅)aminogruppe, ein Metallkation M, eine Gruppe, die unter den folgenden Gruppen (2) bis (7) ausgewählt ist: 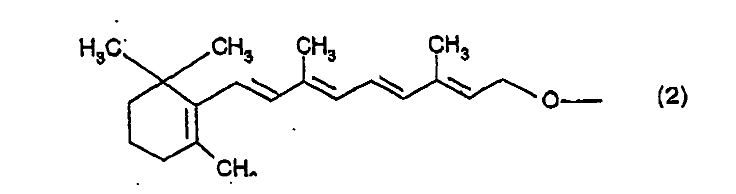 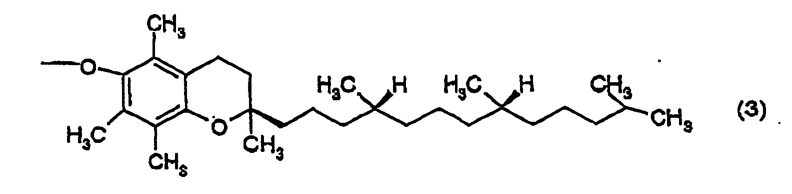 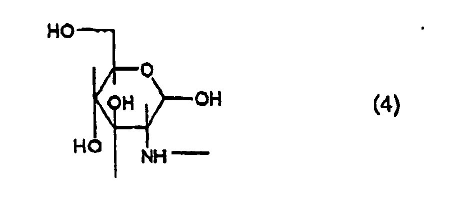 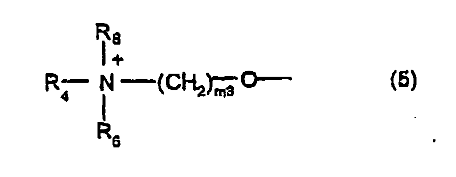 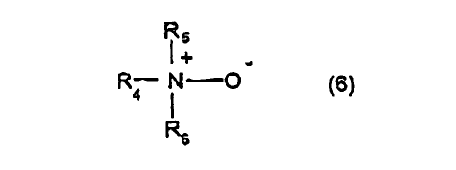 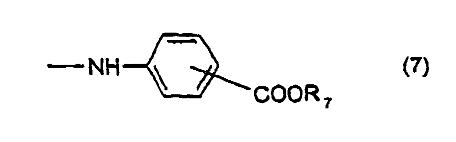
worin bedeuten:
- die Gruppen R₄, R₅ und R₆, die gleich oder verschieden sind, eine unsubstituierte oder mit einer oder mehreren Hydroxygruppen substituierte C₁₋₁₄-Alkylgruppe,
- m₃ eine Zahl von 2 bis 14,
- R₇ ein Wasserstoffatom, ein Metallkation M, eine C₁₋₅-Alkylgruppe oder einen Gruppe der Formel (CH₂)ₘ₂-OT₁ bedeuten, wobei m₂ eine Zahl von 1 bis 4 ist und T₁ die oben angegebenen Bedeutungen aufweist;
(iii) die Gruppen R₁ und R₂, die gleich oder verschieden sind, auch eine Gruppe der folgenden Formel (8) bedeuten können: 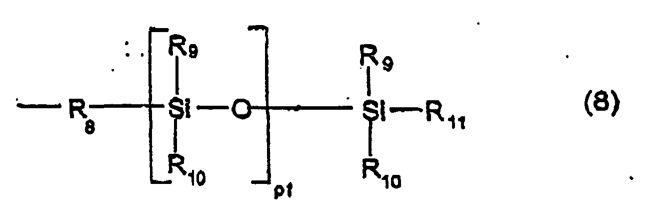 worin bedeuten:
- Ra eine kovalente Bindung, eine geradkettige oder verzweigte C₁₋₄-Alkylgruppe oder einen Rest der Formel -Cₘ₄H₂ₘ₄- oder -Cₘ₄H₂ₘ₄-O-, wobei m₄ eine Zahl von 1 bis 4 ist,
- p₁ eine Zahl von 0 bis 5,
- die Gruppen R₉, R₁₀ und R₁₁, die gleich oder verschieden sind, eine C₁₋₁₈-Alkylgruppe, eine C₁₋₁₈-Alkoxygruppe oder eine Gruppe der Formel: 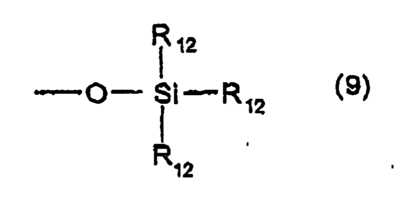
wobei R₁₂ eine C₁₋₅-Alkylgruppe bedeutet,
A₁ einen Rest, der einer der folgenden Formel entspricht: 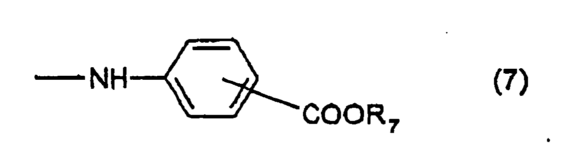 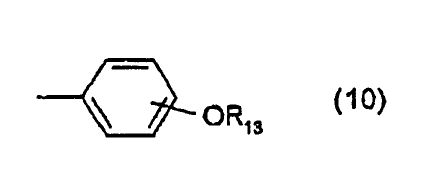 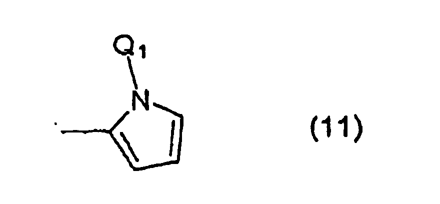 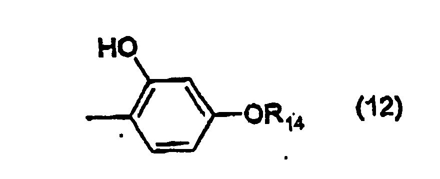 worin bedeuten:
- R₇ die oben angegebenen Bedeutungen,
- R₁₃ ein Wasserstoffatom, eine C₁₋₁₀-Alkylgruppe, eine Gruppe der Formel -(CH₂CHR₁₆-O)ₙ₁R₇, worin n₁ eine Zahl von 1 bis 16 und R₁₆ Wasserstoff oder Methyl bedeutet, oder einen Rest der Formel -CH₂-CH-(OH)-CH₂-OT₁, wobei T₁ die oben angegebenen Bedeutungen aufweist;
- Q₁ eine C₁₋₁₈-Alkylgruppe
- R₁₄ eine Gruppe der folgenden Formel (1), wie sie oben definiert wurde: 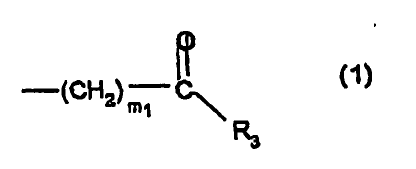
(b) als zweites Filter mindestens eine Verbindung, die pro Molekül mindestens zwei Benzoazolylgruppen aufweist, der folgenden Formel (II): 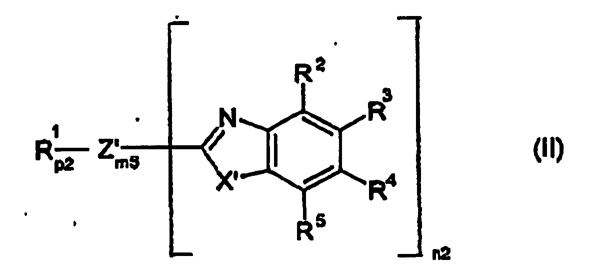 worin:
- Z' ausgewählt ist unter:
(i) olefinischen geradkettigen aliphatischen Kohlenwasserstoffgruppen mit 2 bis 6 Kohlenstoffatomen, die durch eine C₅₋₁₂-Arylgruppe oder eine C₄₋₁₀-Heteroarylgruppe unterbrochen sein können;
(ii) C₅₋₁₂-Arylgruppen, die durch eine olefinische geradkettige aliphatische C₂₋₆-Kohlenwasserstoffgruppe unterbrochen sein können;
(iii) C₃₋₁₀-Heteroarylresten;
wobei die Gruppe Z' mit den Gruppen C₁₋₆-Alkyl, C₁₋₆-Alkoxy, Phenoxy, Hydroxy, Methylendioxy oder Amino, die gegebenenfalls mit einer oder zwei C₁₋₅-Alkylgruppen substituiert sind, substituiert sein kann,
- X' S, O oder NR⁶,
- R¹ Wasserstoff, C₁₋₁₈-Alkyl, C₁₋₄-Alkoxy, C₂₋₁₈-Acyloxy, SO₃Y' oder COOY';
- die Gruppen R², R³, R⁴ und R⁵, die gleich oder verschieden sind, eine Nitrogruppe oder die für R¹ angegebenen Bedeutungen;
- R⁶ Wasserstoff, C₁₋₄-Alkyl oder C₁₋₄-Hydroxyalkyl;
- Y' Wasserstoff, Li, Na, K, NH₄, ½ Ca, ½ Mg, 1/3 Al oder ein Kation, das bei der Neutralisation einer freien Säuregruppe mit einer stickstoffhaltigen organischen Base gebildet wird;
- m₅ 0 oder 1;
- n₂ eine Zahl von 2 bis 6;
- p₂ eine Zahl von 1 bis 4;
- mit der Maßgabe, dass p₂ + n₂ den Wert 6 nicht übersteigt;
wobei die beiden Filter in einem solchen Verhältnis enthalten sind, dass hinsichtlich der erzielten Lichtschutzfaktoren eine synergistische Wirkung erzielt wird.

2. Kosmetische Zusammensetzung zur topischen Anwendung für den Lichtschutz der Haut und/oder der Haare, **dadurch gekennzeichnet, dass** sie in einem kosmetisch akzeptablen Träger enthält:
(a) als erstes Filter mindestens ein spezielles Bis-resorcinyltriazinderivat der Formel (I) nach Anspruch 1;
(b) als zweites Filter mindestens eine Verbindung, die unter den folgenden Verbindungen oder ihren Salzen ausgewählt ist: 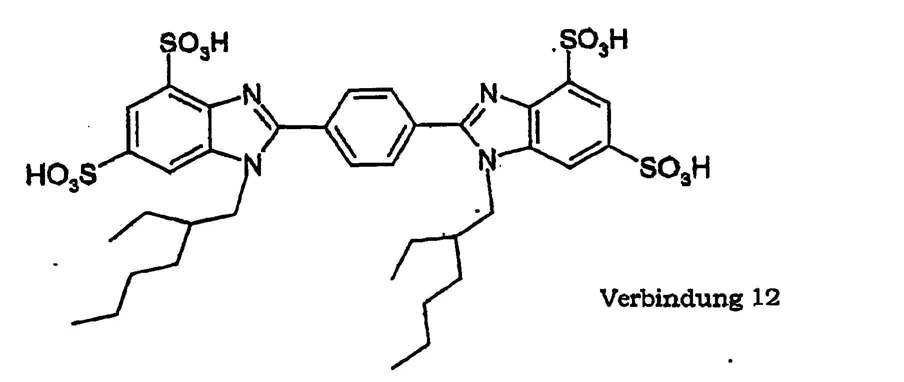 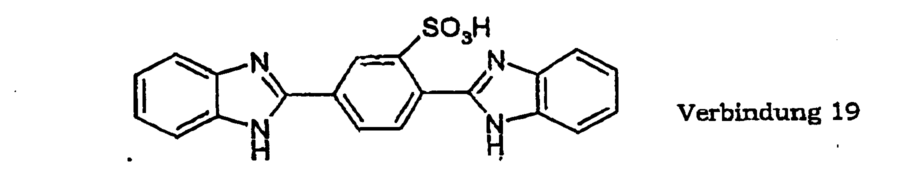 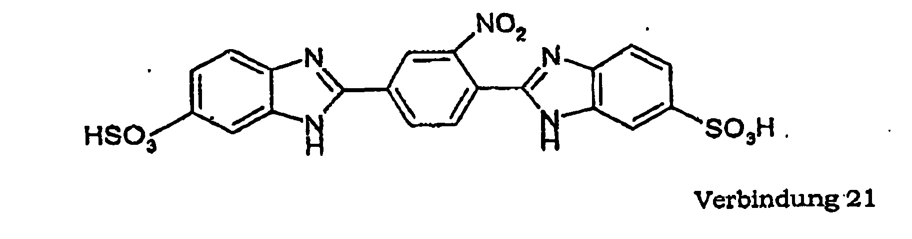 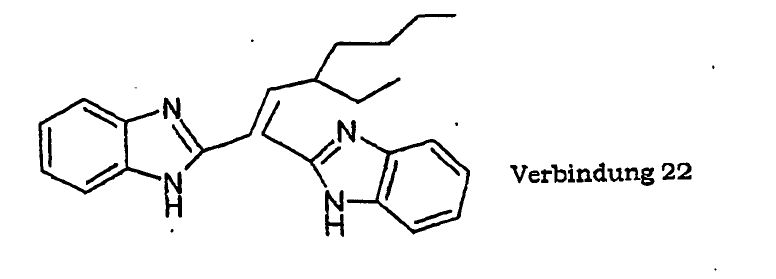 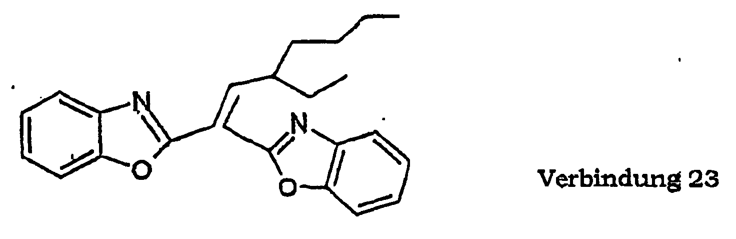 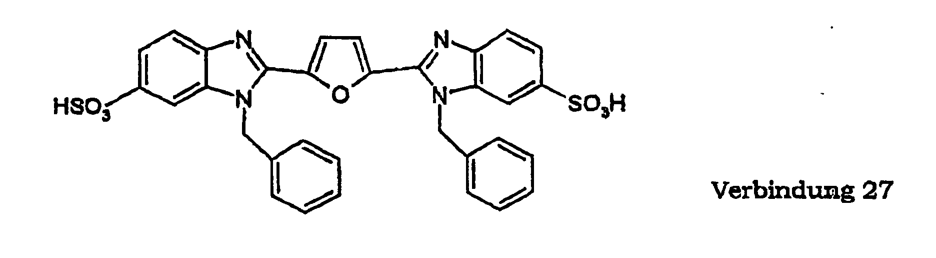 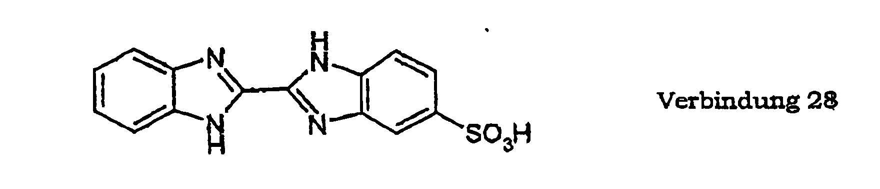 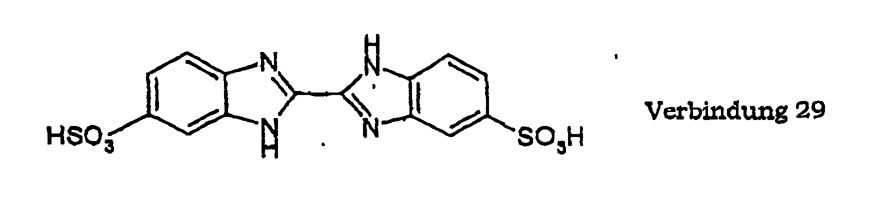 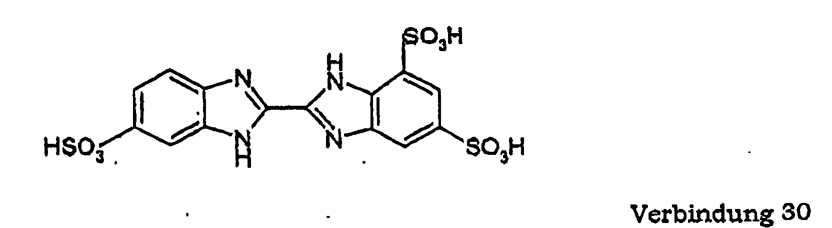 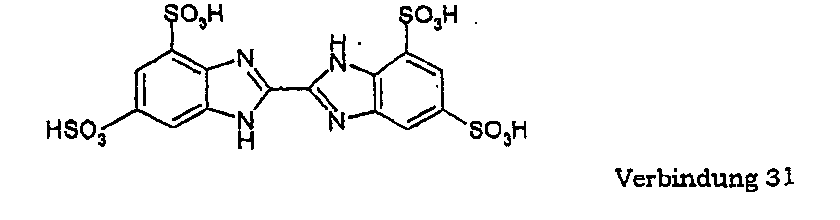 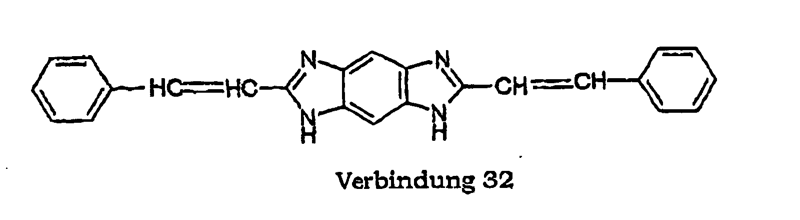 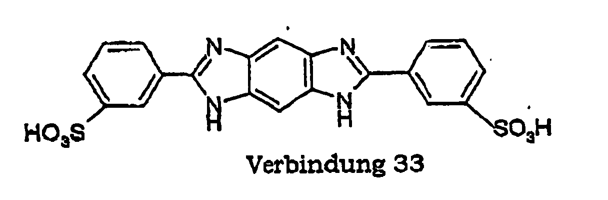 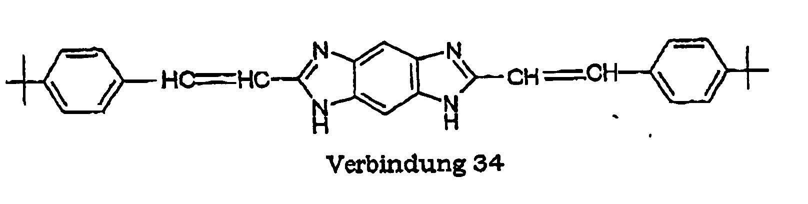 wobei die beiden Filter in einem solchen Verhältnis enthalten sind, dass hinsichtlich der erzielten Lichtschutzfaktoren eine synergistische Wirkung erzielt wird.

3. Zusammensetzung nach Anspruch 1 oder 2, **dadurch gekennzeichnet, dass** die Verbindung der Formel (I) ausgewählt ist unter:
(1) Verbindungen, worin A₁ p-Methoxyphenyl oder p-Ethoxyphenyl und die Gruppen R₁ und R₂, die gleich oder verschieden sind, eine Gruppe der folgenden Struktur bedeuten: 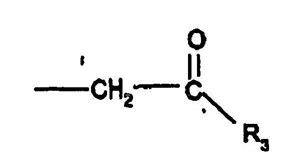 worin R₃ ausgewählt ist unter:
- *t*-Butyloxy,
- OH,
- OM, wobei M ein Alkali- oder Erdalkalikation oder ein Kation ist, das unter Kupfer, Magnesium oder Zink ausgewählt ist,
- einer Gruppe der folgenden Struktur: 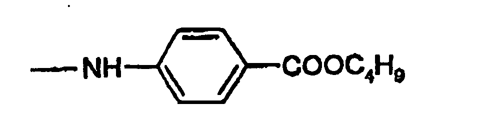
- einer Gruppe der Struktur O⁻N⁺(CH₂CH₂OH)₃,
- einer Gruppe der folgenden Struktur: 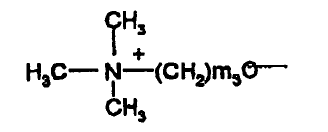
wobei m₃ im Bereich von 2 bis 14 liegt,
- einer Gruppe der folgenden Struktur: 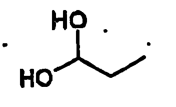
- einer Gruppe der Struktur: 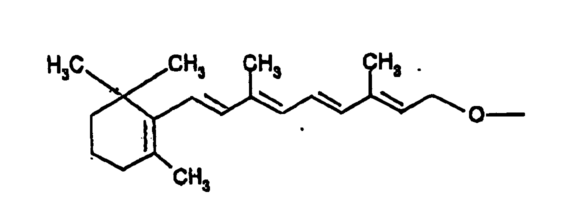
(2) Bis-resorcinyl-triazinderivaten der Formel (I), worin A₁ p-Hydroxyphenyl und die Gruppen R₁ und R₂ gleichzeitig eine Gruppe der folgenden Struktur bedeuten:

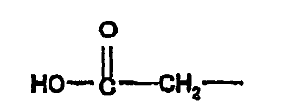

4. Zusammensetzung nach Anspruch 3, **dadurch gekennzeichnet, dass** die Verbindung der Formel (I) ausgewählt ist unter:
- 2,4-Bis{[4-(2-ethyl-hexyloxy)-2-hydroxy]-phenyl}-6-(4-methoxyphenyl)-1,3,5-triazin;
- 2,4-Bis{[4-(3-(2-propyloxy)-2-hydroxy-propyloxy)-2-hydroxy]-phenyl}-6-(4-methoxy-phenyl)-1,3,5-triazin;
- 2,4-Bis{[4-(2-ethyl-hexyloxy)-2-hydroxy]-phenyl}-6-[4-(2-methoxyethyl-carboxy)-phenylamino)-1,3,5-triazin;
- 2,4-Bis{[4-tris(trimethylsiloxy-silylpropyloxy)-2-hydroxy]-phenyl}-6-(4-methoxyphenyl)-1,3,5-triazin;
- 2,4-Bis{[4-(2"-methylpropenyloxy)-2-hydroxy]-phenyl}-6-(4-methoxyphenyl)-1,3,5-triazin;
- 2,4-Bis{[4-(1',1',1',3',5',5',5'-heptamethyltrisiloxy-2"-methylpropyloxy)-2-hydroxy]-phenyl}-6-(4-methoxyphenyl)-1,3,5-triazin;
- 2,4-Bis{[4-(3-(2-propyloxy)-2-hydroxy-propyloxy]-2-hydroxy]-phenyl}-6-([4-ethylcarboxy)-phenylamino)-1,3,5-triazin;
- 2,4-Bis{[4-(2-ethyl-hexyloxy)-2-hydroxy]-phenyl}-6-(1-methylpyrrol-2-yl)-1,3,5-triazin.

5. Zusammensetzung nach Anspruch 4, **dadurch gekennzeichnet, dass** die Verbindung der Formel (I) ausgewählt ist unter:
- 2,4-Bis{[4-(2-ethyl-hexyloxy)-2-hydroxy]-phenyl}-6-(4-methoxyphenyl)-1,3,5-triazin;
- 2,4-Bis{[4-tris(trimethylsiloxy-silylpropyloxy)-2-hydroxy]-phenyl}-6-(4-methoxyphenyl)-1,3,5-briazin;
- 2,4-Bis{(4-(1',1',1',3',5',5',5'-heptamethyltrisiloxy-2"-methylpropyloxy)-2-hydroxy]-phenyl}-6-(4-methoxyphenyl)-1,3,5-triazin.

6. Zusammensetzung nach einem der vorhergehenden Ansprüche, **dadurch gekennzeichnet, dass** die Konzentration des Bis-resorcinyl-triazinderivats im Bereich von 0,1 bis 15 Gew.-%, bezogen auf das Gesamtgewicht der Zusammensetzung, liegt.

7. Zusammensetzung nach Anspruch 6, **dadurch gekennzeichnet, dass** die Konzentration im Bereich von 0,2 bis 10 Gew.-%, bezogen auf das Gesamtgewicht der Zusammensetzung, liegt.

8. Zusammensetzung nach einem der Ansprüche 1 und 3 bis 7, wobei die Gruppe Z' unter den folgenden Gruppen ausgewählt ist:
-CH=CH-
-CH=CH-CH=CH- 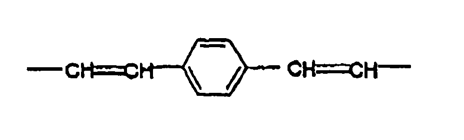 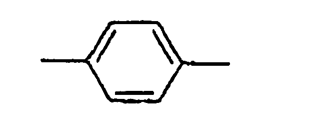 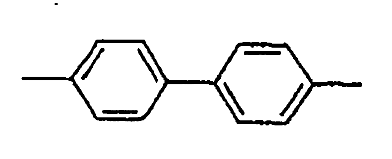 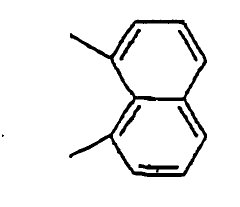 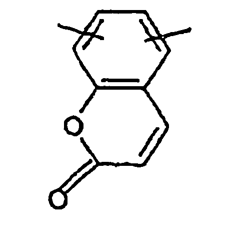 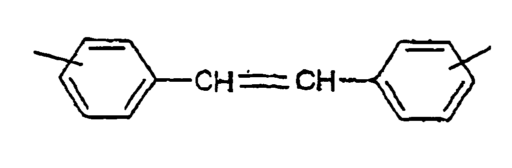 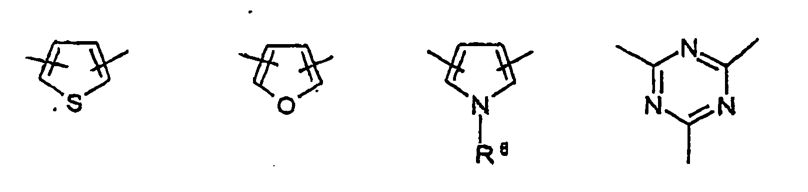
wobei R⁶ die in Anspruch 1 angegebenen Bedeutungen aufweist.

9. Zusammensetzung nach einem der Ansprüche 1 und 3 bis 7, wobei die Verbindung der Formel (II) unter den folgenden Verbindungen und ihren Salzen ausgewählt ist:

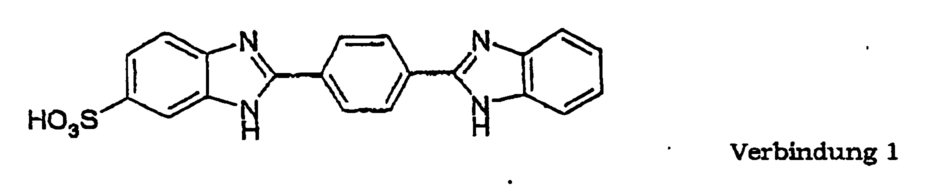

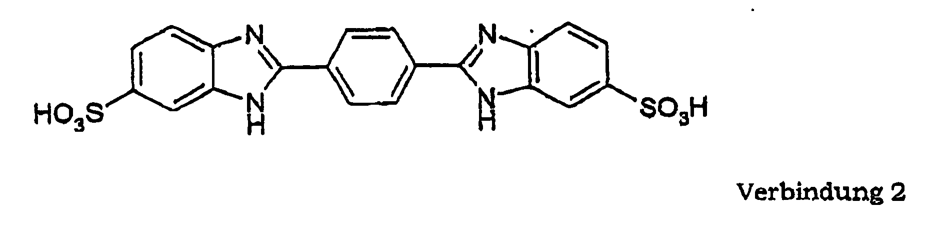

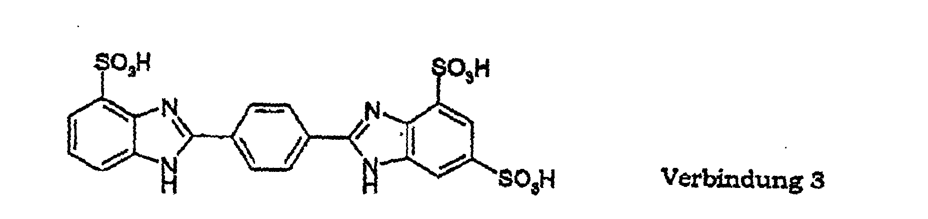

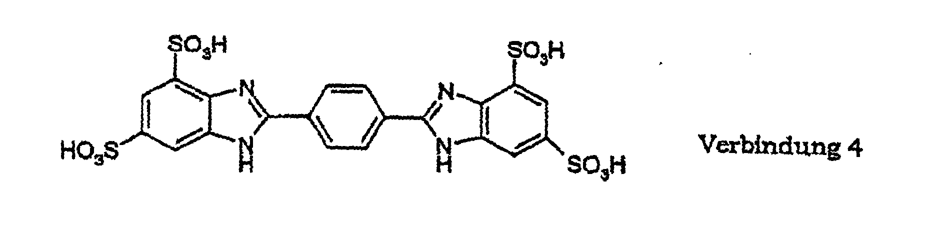

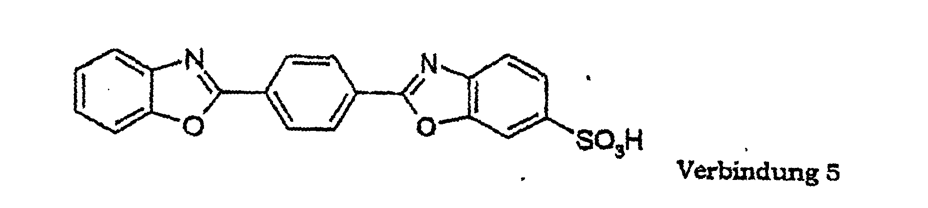

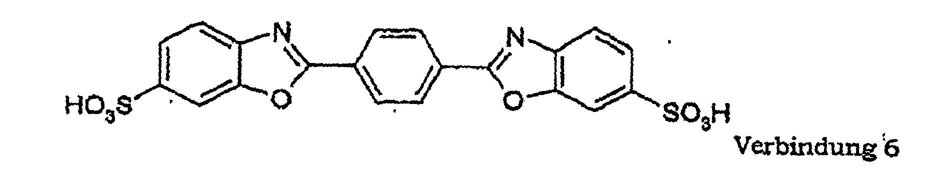

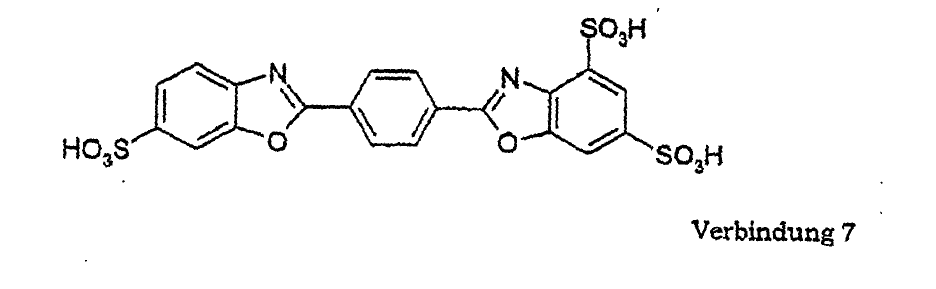

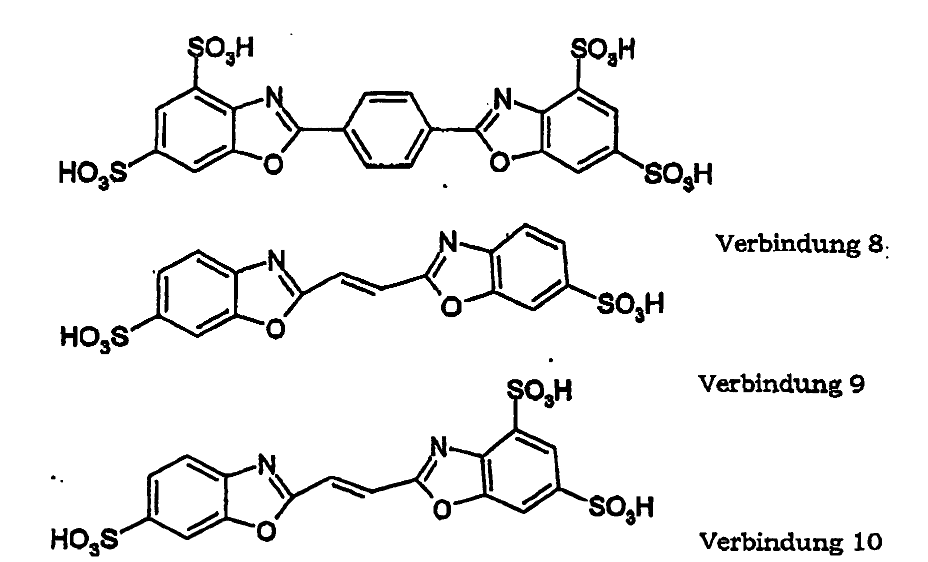

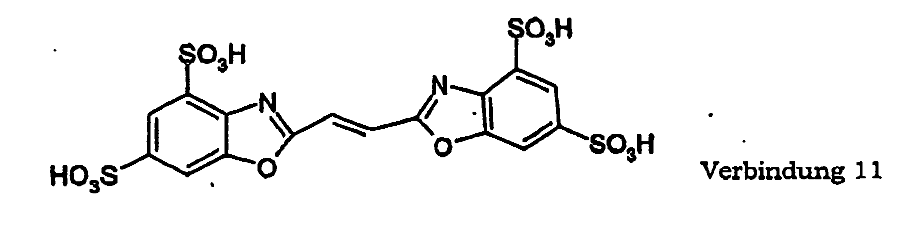

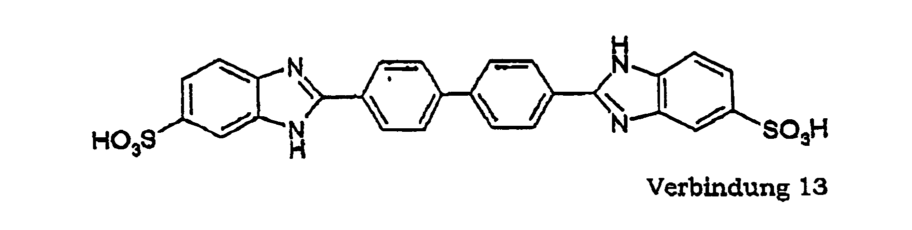

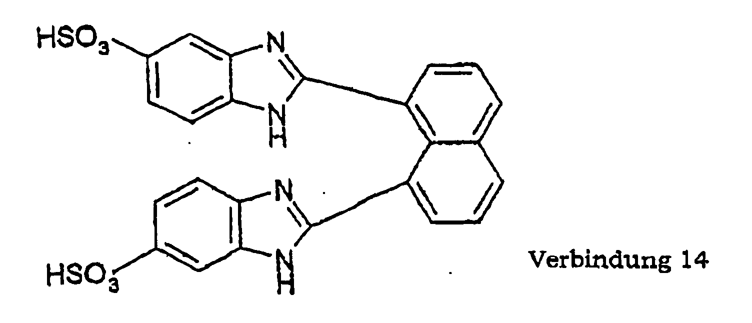

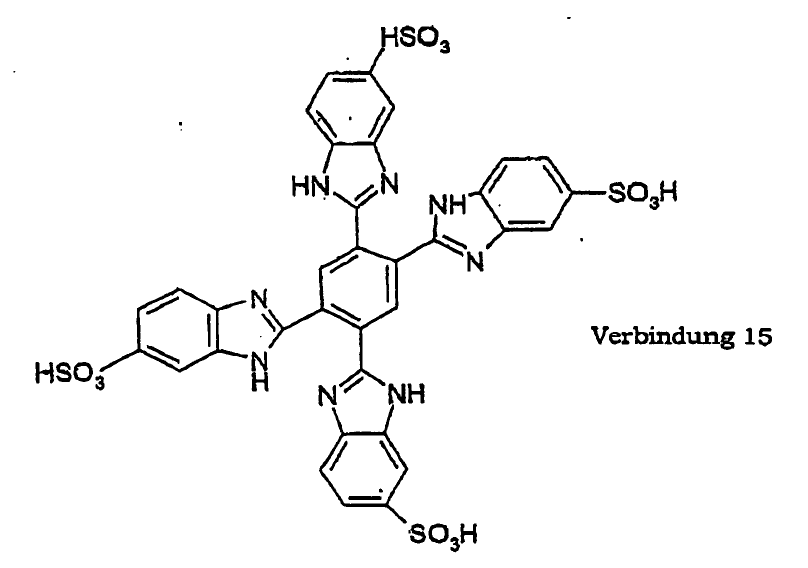

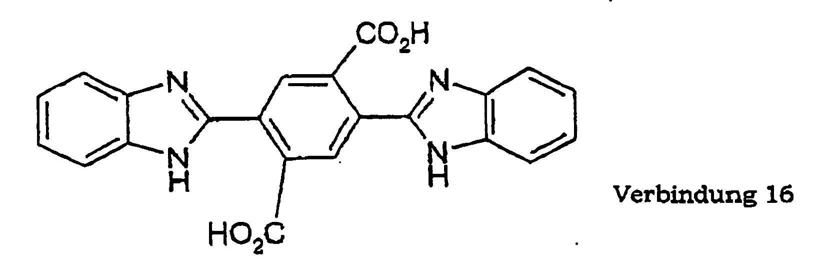

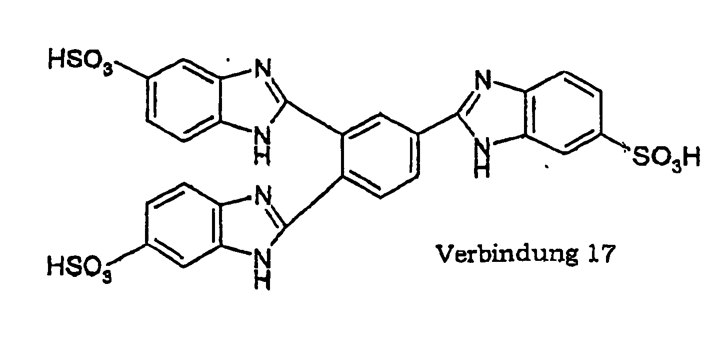

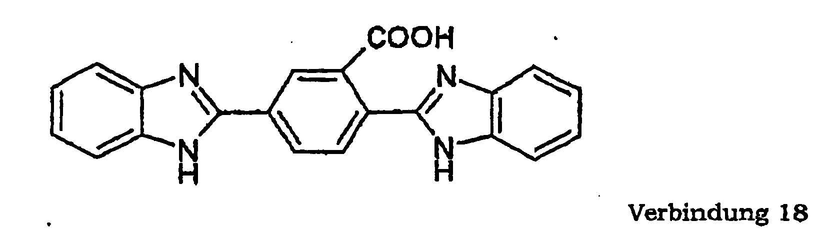

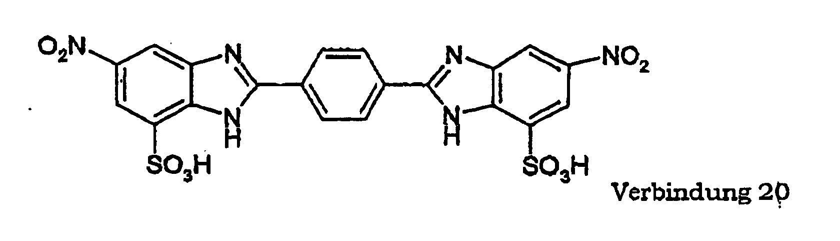

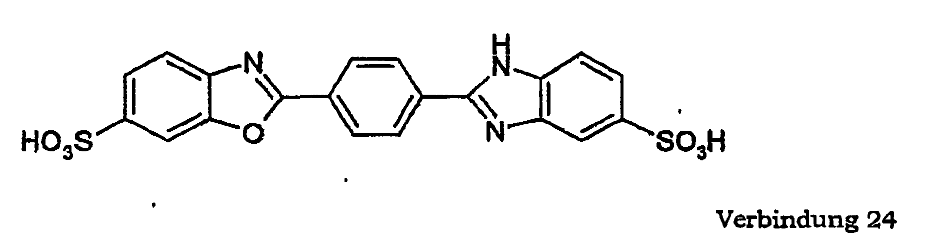

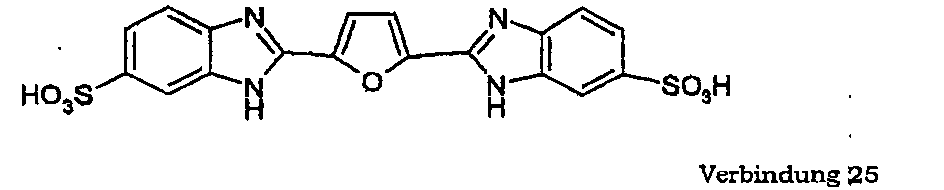

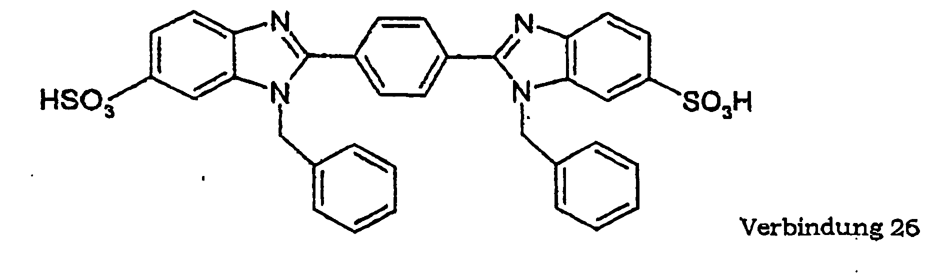

10. Zusammensetzung nach Anspruch 9, wobei es sich bei der Verbindung der Formel (II) um die Phenylen-1,4-bis-benzimidazolyl--3,3',5,5'-tetrasulfonsäure der folgenden Struktur oder eines ihrer Salze handelt:

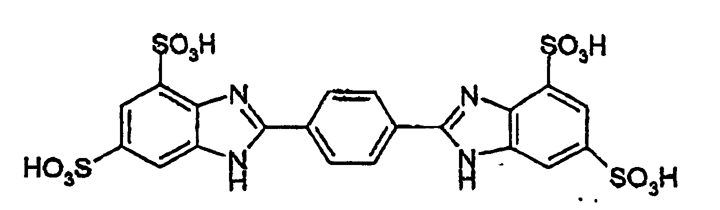

11. Zusammensetzung nach einem der Ansprüche 1 bis 10, **dadurch gekennzeichnet, dass** die in einem der Ansprüche 1 und 3 bis 10 definierte(n) Verbindung(en) der Formel (II) oder die Verbindung(en) nach Anspruch 2 in einer Konzentration von 0,1 bis 15 Gew.-%, bezogen auf das Gesamtgewicht der Zusammensetzung, enthalten sind.

12. Zusammensetzung nach Anspruch 11, **dadurch gekennzeichnet, dass** die Konzentration im Bereich von 0,2 bis 10 Gew.-%, bezogen auf das Gesamtgewicht der Zusammensetzung, liegt.

13. Zusammensetzung nach einem der Ansprüche 1, bis 12, **dadurch gekennzeichnet, dass** der kosmetisch akzeptable Träger als Emulsion von Öl-in-Wasser-Typ vorliegt.

14. Zusammensetzung nach einem der Ansprüche 1 bis 13, **dadurch gekennzeichnet, dass** sie ferner ein oder mehrere, ergänzende, hydrophile oder lipophile organische Filter enthält, die im UV-A-Bereich und/oder UV-B-Bereich wirksam sind und die von dem genannten ersten und zweiten Filter verschieden sind.

15. Zusammensetzung nach Anspruch 14, **dadurch gekennzeichnet, dass** die ergänzenden organischen Filter unter den Zimtsäurederivaten, Salicylsäurederivaten, Campherderivaten, Triazindenvaten, die von den in den vorhergehenden Ansprüchen definierten Triazinderivaten verschieden sind, Benzimidazolderivaten, die nonderivaten, Dibenzoylmethanderivaten, β,β'-Diphenylacrylat-derivaten, p-Aminobenzoesäurederivaten, polymeren Filtern und Siliconfiltern ausgewählt sind.

16. Zusammensetzung nach einem der Ansprüche 1 bis 15, **dadurch gekennzeichnet, dass** sie als ergänzende UV-Lichtschutzmittel ferner gegebenenfalls umhüllte Pigmente oder Nanopigmente von Metalloxiden enthält.

17. Zusammensetzung nach Anspruch 16, **dadurch gekennzeichnet, dass** die Pigmente oder Nanopigmente unter den gegebenenfalls umhüllten Oxiden von Titan, Zink, Eisen, Zirconium oder Cer und deren Gemischen ausgewählt sind.

18. Zusammensetzung nach einem der Ansprüche 1 bis 17, **dadurch gekennzeichnet, dass** sie außerdem mindestens ein Bräunungsmittel und/ oder Mittel zur künstlichen Braunfärbung der Haut enthält.

19. Zusammensetzung nach einem der Ansprüche 1 bis 18, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Antioxidantien, Trübungsmitteln, Stabilisatoren, Emollientien, Hydroxysäuren, Schaumverhütungsmitteln, Hydratisierungsmitteln, Vitaminen, Parfums, Konservierungsmitteln, grenzflächenaktiven Stoffen, Füllstoffen, Maskierungsmitteln, Polymeren, Treibmitteln, Alkalisierungsmitteln, Ansäuerungsmitteln und Farbmitteln ausgewählt ist.

20. Zusammensetzung nach einem der Ansprüche 1 bis 19, **dadurch gekennzeichnet, dass** es sich um Zusammensetzungen, die die menschliche Epidermis schützen, oder Sonnenschutzmittel handelt und diese als nichtionische Vesikeldispersionen, Emulsionen, insbesondere Öl-in-Wasser-Emulsionen, Cremes, Milche, Gele, Gelcremes, Suspensionen, Dispersionen, Puder, feste Stifte, Schäume und Sprays vorliegen.

21. Zusammensetzung nach einem der Ansprüche 1 bis 20, **dadurch gekennzeichnet, dass** es sich um Zusammensetzungen zum Schminken der Wimpern, Augenbrauen oder der Haut handelt, und **dadurch**, dass sie in fester oder pastöser, wasserfreier oder wässriger Form, als Emulsionen, Suspensionen oder Dispersionen vorliegen.

22. Zusammensetzung nach einem der Ansprüche 1 bis 20, **dadurch gekennzeichnet, dass** es sich um Zusammensetzungen zum Schutz der Haare gegen UV-Strahlung handelt, und **dadurch**, dass sie als Haarwaschmittel, Lotionen, Gele, Emulsionen oder nichtionische Vesikeldispersionen vorliegen.

23. Verwendung der Zusammensetzung nach einem der Ansprüche 1 bis 22 für die Herstellung von kosmetischen Produkten zum Schutz der Haut oder der Haare gegen UV-Strahlung und insbesondere gegen Sonnenlicht.

24. Verfahren zur kosmetischen Behandlung zum Schutz der Haut und/oder der Haare gegen UV-Strahlung, insbesondere Sonnenlicht, **dadurch gekennzeichnet, dass** auf diese eine wirksame Menge einer Zusammensetzung nach einem der Ansprüche 1 bis 22 aufgetragen wird.

## Claims

1. Cosmetic composition for topical use, for the photoprotection of the skin and/or hair, **characterized in that** it comprises, in a cosmetically acceptable vehicle:
(a), as first screening agent, at least one bisresorcinyltriazine derivative of following formula (I): 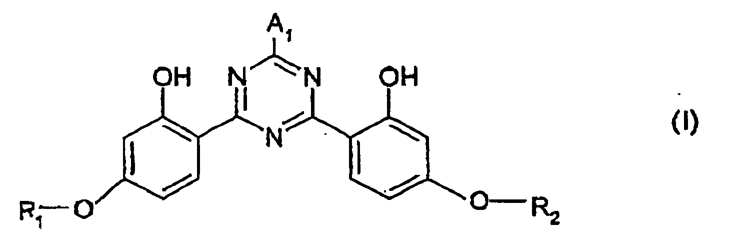
in which:
(i) the R₁ and R₂ radicals, which are identical or different, denote a C₃-C₁₈ alkyl radical, a C₂-C₁₈ alkenyl radical or a residue of formula -CH₂-CH(OH)-CH₂-OT₁ where T₁ is a hydrogen atom or a C₁-C₈ alkyl radical;
(ii) the R₁ and R₂ radicals, which are identical or different, can also denote a residue of following formula (1): 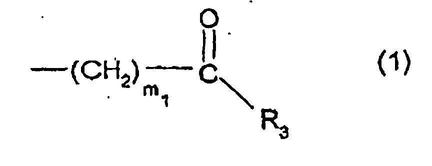 in which:
- m₁ is a number from 1 to 3;
- R₃ denotes a hydroxyl group; a C₁-C₅ alkyl radical which is unsubstituted or substituted by one or more hydroxyl groups; a C₁-C₅ alkoxy radical; an amino group; a mono- or di(C₁-C₅)alkylamino radical; a metal cation M; or a residue chosen from one of the following formulae (2) to (7) : 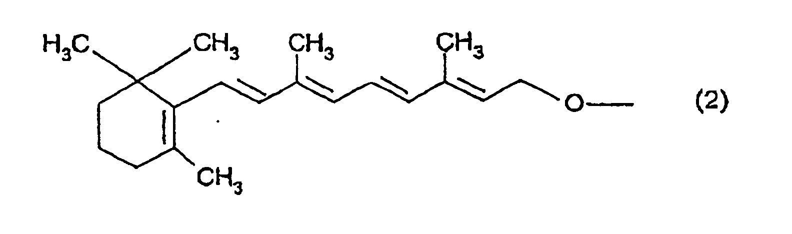 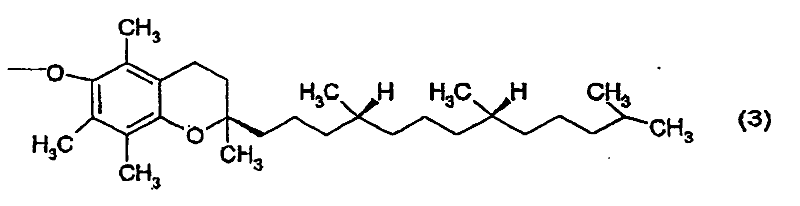 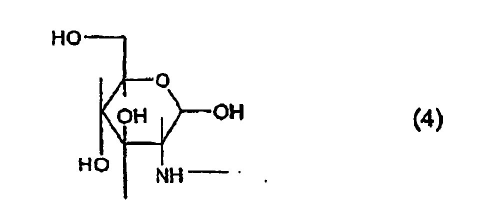 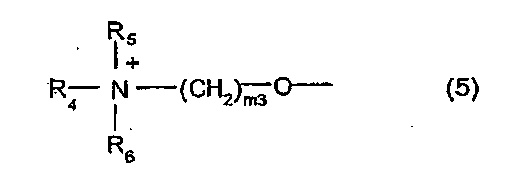 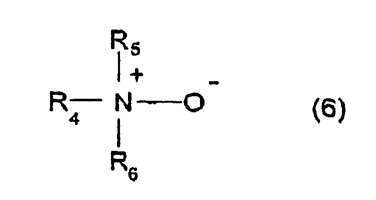 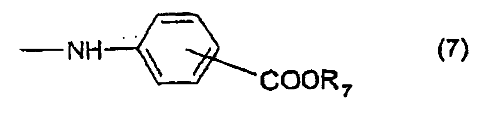
in which:
- the R₄, R₅ and R₆ radicals, which are identical or different, denote a C₁-C₁₄ alkyl radical which is unsubstituted or substituted by one or more hydroxyl groups;
- m₃ is a number from 2 to 14;
- R₇ represents a hydrogen atom, a metal cation M, a C₁-C₅ alkyl radical or a residue of formula -(CH₂)_{m₂}-OT₁ where m₂ is a number from 1 to 4 and T₁ has the same meaning indicated above;
(iii) the R₁ and R₂ radicals, which are identical or different, can also denote a residue of following formula (8): 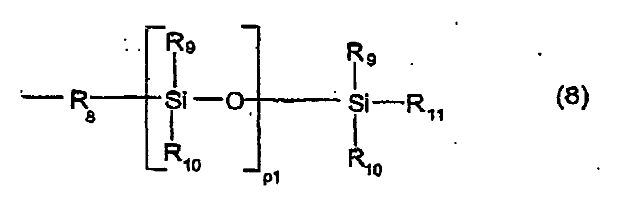
in which:
- R₈ denotes a covalent bond, a linear or branched C₁-C₄ alkyl, radical or a residue of formula -C_{m₄}H_{2m₄}- or
- C_{m₄}H_{2m₄}-O- where m₄ is a number from 1 to 4;
- p₁ is a number from 0 to 5;
- the R₉, R₁₀ and R₁₁ radicals, which are identical or different, denote a C₁-C₁₈ alkyl radical, a C₁-C₁₈ alkoxy radical or a residue of formula: 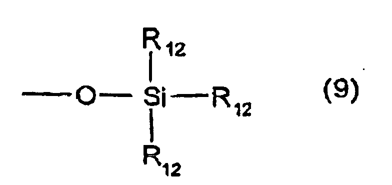
where R₁₂ is a C₁-C₅ alkyl radical;
- A₁ denotes a residue corresponding to one of the following formulae: 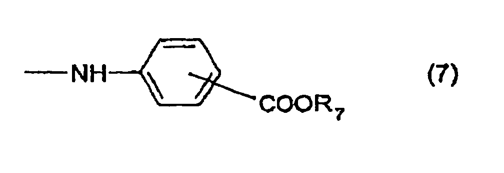 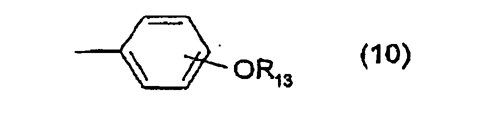 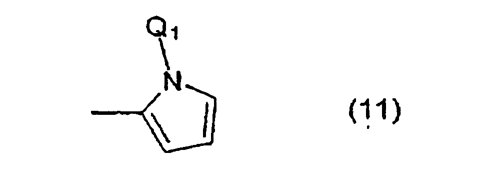 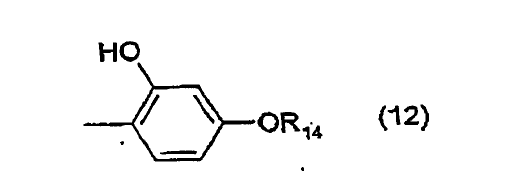
in which:
- R₇ has the same meanings indicated above;
- R₁₃ denotes a hydrogen atom, a C₁-C₁₀ alkyl radical, a radical of formula -(CH₂CHR₁₆-O)_{n₁}R₇ where n₁ is a number from 1 to 16 and R₁₆ is hydrogen or methyl, or a residue of structure -CH₂-CH(OH)-CH₂OT₁ with T₁ having the same meaning indicated above;
- Q₁ is a C₁-C₁₈ alkyl radical;
- R₁₄ is a radical corresponding to the formula (1): 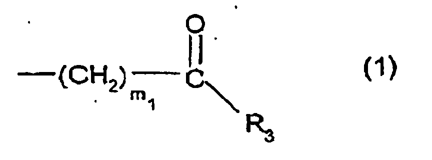
and (b), as second screening agent, at least one compound comprising, per molecule, at least two benzazolyl groups of following formula (II): 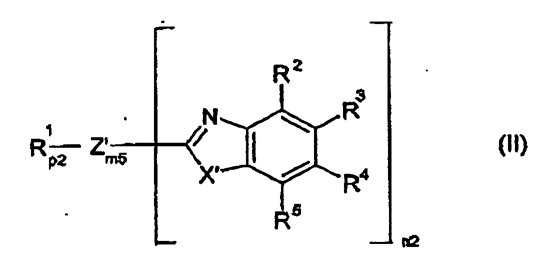 in which:
- Z' is chosen from the group consisting of:
(i) an olefinic linear aliphatic C₂-C₆ hydrocarbonaceous radical which can be interrupted by a C₅-C₁₂ aryl group or a C₄-C₁₀ heteroaryl;
(ii) a C₅-C₁₅ aryl group which can be interrupted by an olefinic linear aliphatic C₂-C₆ hydrocarbonaceous radical;
(iii) a C₃-C₁₀ heteroaryl group, it being possible for the said Z' radical to be substituted by C₁-C₆ alkyl, C₁-C₆ alkoxy, phenoxy, hydroxyl, methylenedioxy or amino radicals, the amino radicals optionally being substituted by one or 2 C₁-C₅ alkyl radicals;
- X' denotes S, O or NR⁶;
- R¹ denotes hydrogen, a C₁-C₁₈ alkyl, a C₁-C₄ alkoxy, a C₅-C₁₅ aryl, a C₂-C₁₈ acyloxy, SO₃Y' or COOY';
- the R², R³, R⁴ and R⁵ radicals, which are identical or different, denote a nitro group or have the same meanings as R¹;
- R⁶ denotes hydrogen, a C₁-C₄ alkyl or a C₁-C₄ hydroxyalkyl;
- Y' denotes hydrogen, Li, Na, K, NH₄, 1/2Ca, 1/2Mg, 1/3Al or a cation resulting from the neutralization of a free acid group by a nitrogenous organic base;
- m₅ is 0 or 1;
- n₂ is a number from 2 to 6;
- p₂ is a number from 1 to 4;
- with the proviso that p₂ + n₂ does not exceed the value 6; the said first and second screening agents being present in a proportion producing a synergistic activity with respect to the sun protection factors conferred.

2. Cosmetic compostion for topical use, for the photoprotection of the skin and/or hair, **characterized in that** it comprises, in a cosmetically acceptable vehicle:
(a) as first screening agent, at least one bisresorcinyltriazine derivative of formula (I) as defined in Claim 1;
(b) as second screening agent, at least one compound chosen from the following compounds or one of their salts: 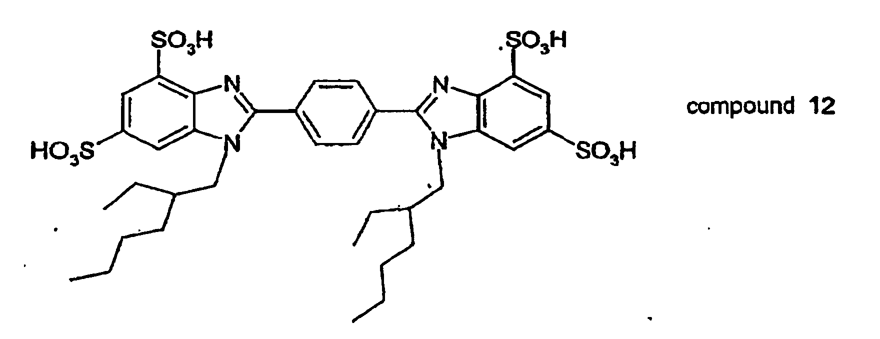 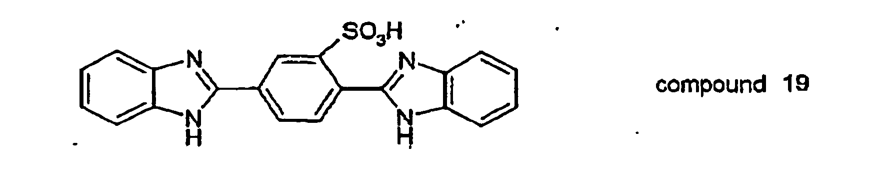 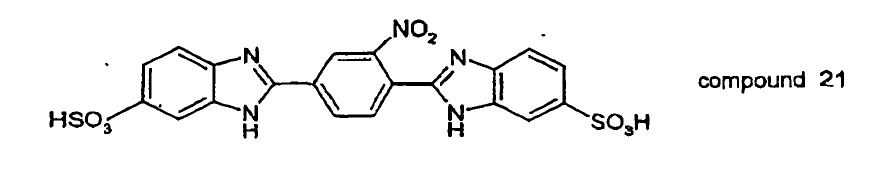 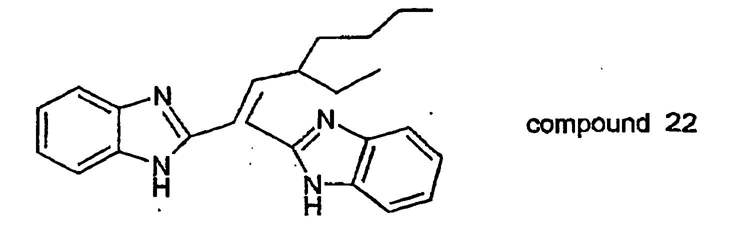 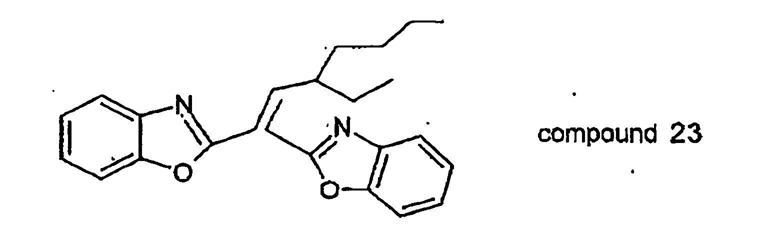 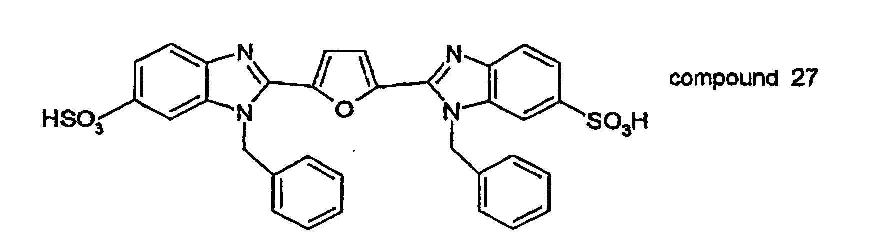 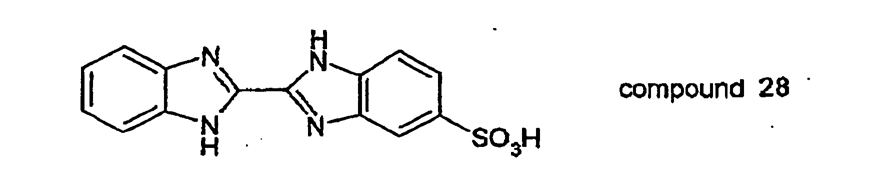 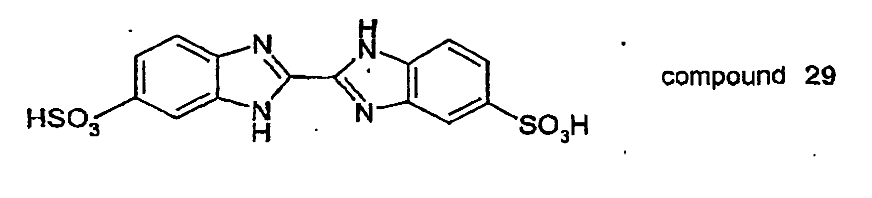 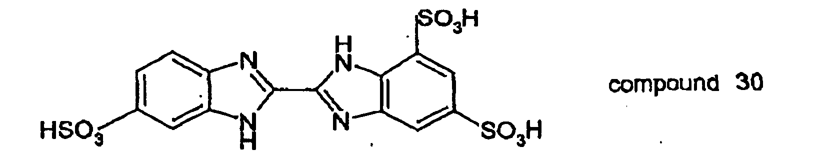 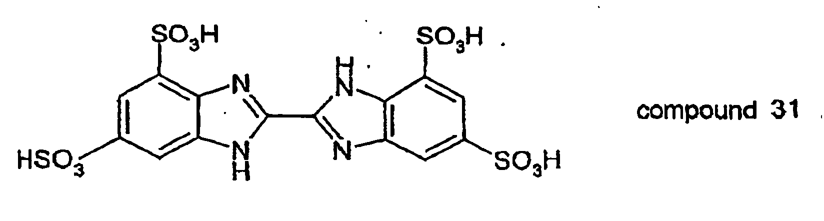 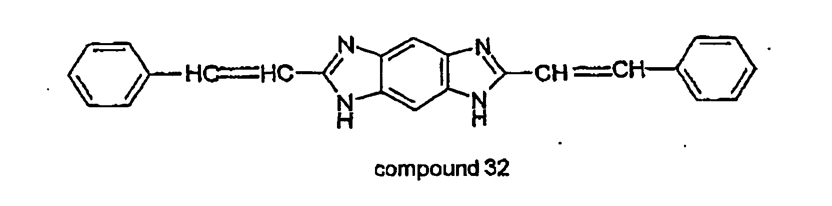 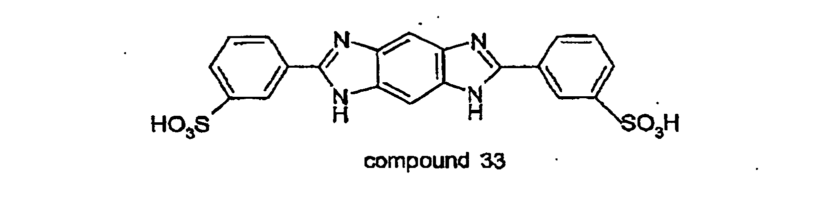 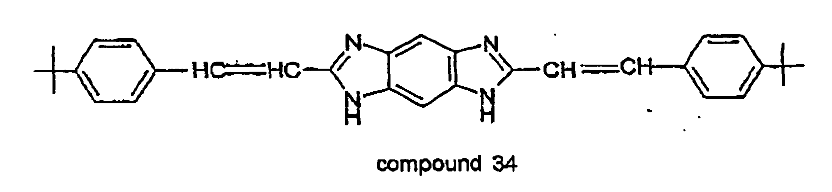 the said first and second screening agents being present in a proportion producing a synergistic activity with respect to the sun protection factors conferred.

3. Composition according to Claim 1 or 2, **characterized in that** the compound of formula (I) is chosen from:
(1) those in which the A₁ radical denotes para-methoxyphenyl or para-ethoxyphenyl and the R₁ and R₂ radicals, which are identical or different, denote a radical with the structure: 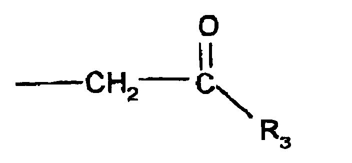 in which structure R₃ is chosen from:
- tert-butyloxy;
- OH;
- OM, where M is an alkali metal or alkaline earth metal cation or a cation chosen from copper, magnesium or zinc;
- a group with the structure: 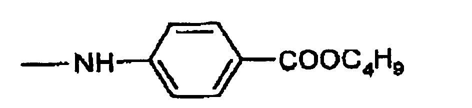
- a group with the structure O⁻N⁺(CH₂CH₂OH)₃;
- a group with the structure: 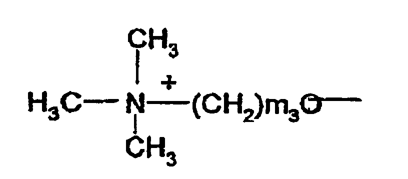 with m₃ varying from 2 to 14;
- a group with the structure: 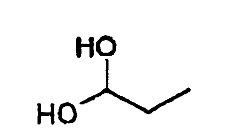
- a group with the structure: 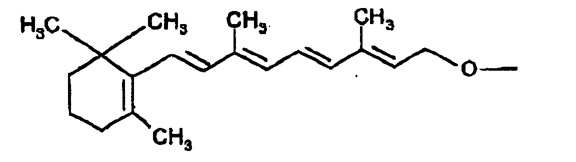
(2) the bisresorcinyltriazine compound corresponding to the formula (I) in which the A₁ radical denotes para-hydroxyphenyl and the R₁ and R₂ radicals simultaneously denote a group with the structure:

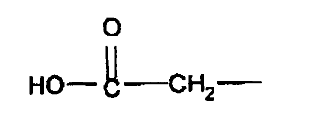

4. Composition according to Claim 3, **characterized in that** the compound of formula (I) is chosen from the group consisting of:
- 2,4-bis{[4-(2-ethylhexyloxy)-2-hydxoxy]phenyl}-6-(4-methoxyphenyl)-1,3,5-triazine;
- 2,4-bis{[4-(3-(2-propyloxy)-2-hydroxypropyloxy)-2-hydroxy]phenyl}-6-(4-methoxyphenyl)-1,3,5-triazine;
- 2,4-bis{[4-(2-ethylhexyloxy)-2-hydroxy]phenyl}-6-[4-(2-methoxyethylcarboxyl)phenylamino]-1,3,5-triazine;
- 2,4-bis{[4-(tris(trimethylsiloxy)silylpropyloxy)-2-hydroxy]phenyl}-6-(4-methoxyphenyl)-1,3,5-triazine;
- 2,4-bis{[4-(2"-methylpropenyloxy)-2-hydroxy]phenyl}-6-(4-methoxyphenyl)-1,3,5-triazine;
- 2,4-bis{[4-(1',1',1',3',5',5',5'-heptamethyltrisiloxy-2"-methylpropyloxy)-2-hydroxy]phenyl}-6-(4-methoxyphenyl)-1,3,5-triazine;
- 2,4-bis{[4-(3-(2-propyloxy)-2-hydroxypropyloxy)-2-hydroxy]phenyl}-6-[4-(ethylcarboxyl)phenylamino]-1,3,5-triazine;
- 2,4-bis{[4-(2-ethylhexyloxy)-2-hydroxy]phenyl}-6-(1-methylpyrrol-2-yl)-1,3,5-triazine.

5. Composition according to Claim 4, **characterized in that** the compound of formula (I) is chosen from the group consisting of:
- 2,4-bis{[4-(2-ethylhexyloxy)-2-hydroxy]phenyl}-6-(4-methoxyphenyl)-1,3,5-triazine;
- 2,4-bis{[4-(tris(trimethylsiloxy)silylpropyloxy)-2-hydroxy]phenyl}-6-(4-methoxyphenyl)-1,3,5-triazine;
- 2,4-bis{[4-(1',1',1',3',5',5',5'-heptamethyltrisiloxy-2"-methylpropyloxy)-2-hydroxy]phenyl}-6-(4-methoxyphenyl)-1,3,5-triazine.

6. Composition according to any one of the preceding claims, **characterized in that** the concentration of bisresorcinyltriazine derivative is between 0.1 and 15% by weight with respect to the total weight of the composition.

7. Composition according to Claim 6, **characterized in that** the said concentration is between 0.2 and 10% by weight with respect to the total weight of the composition.

8. Composition according to any one of Claims 1 and 3 to 7, where the Z' radical is chosen from the group consisting of the following groups:
-CH=CH-,
-CH=CH-CH=CH- 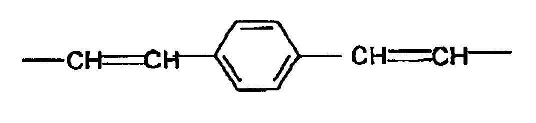 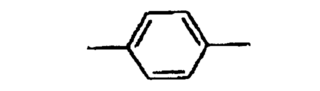 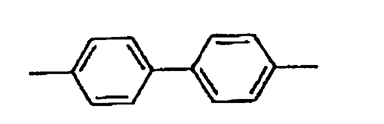 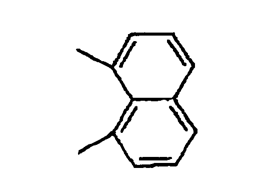 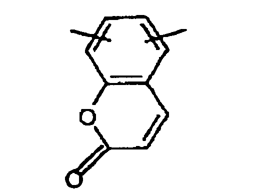 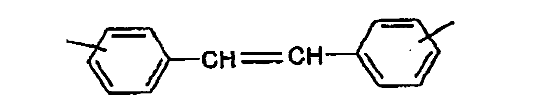 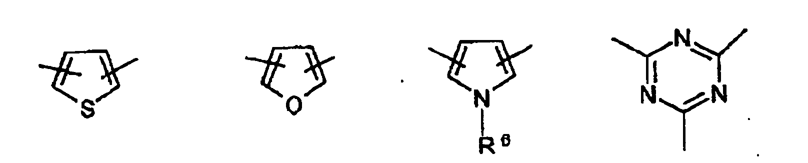
with R⁶ having the same meaning indicated in Claim 1.

9. Composition according to any one of Claims 1 and 3 to 7, where the compound of formula (II) is chosen from the following compounds or one of their salts:

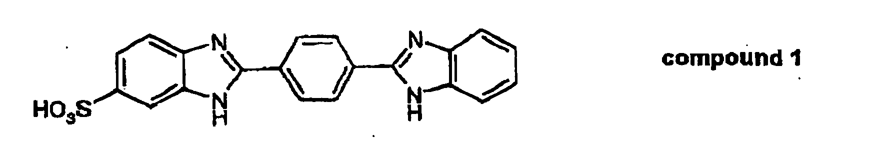

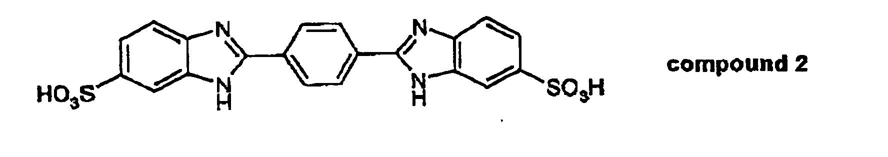

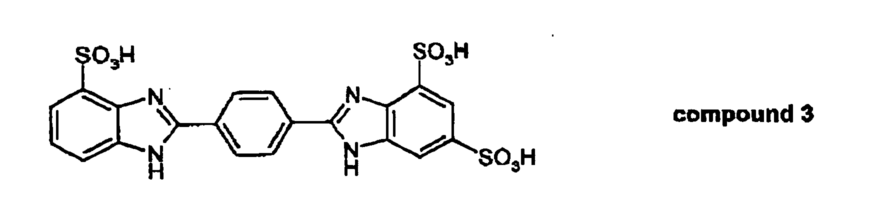

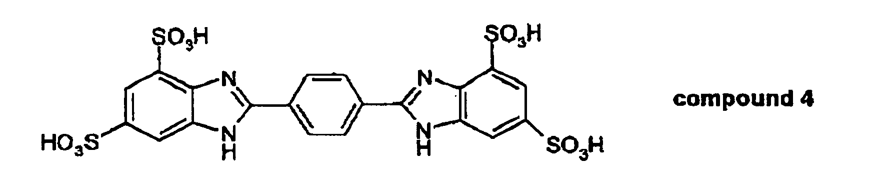

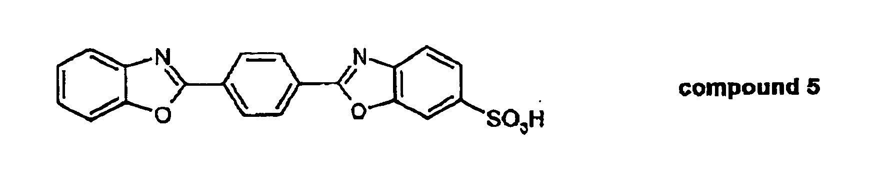

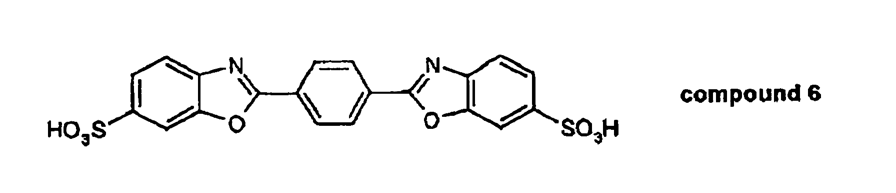

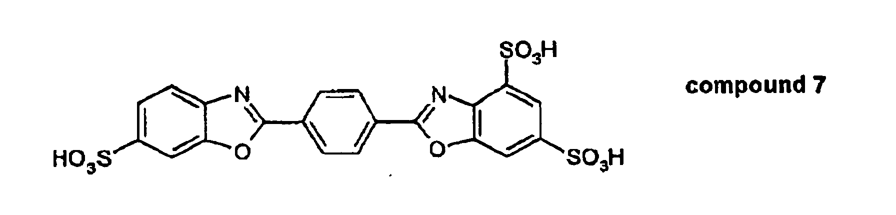

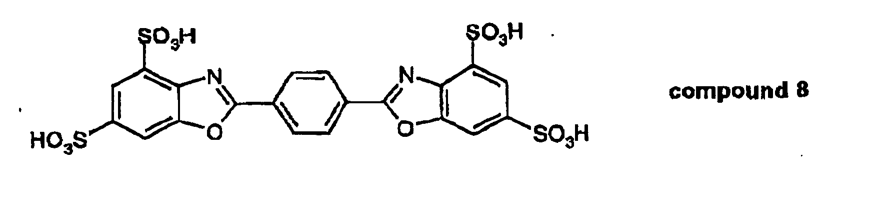

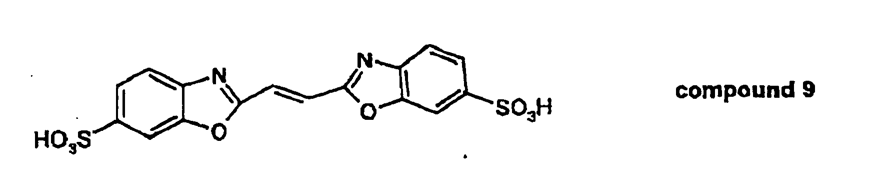

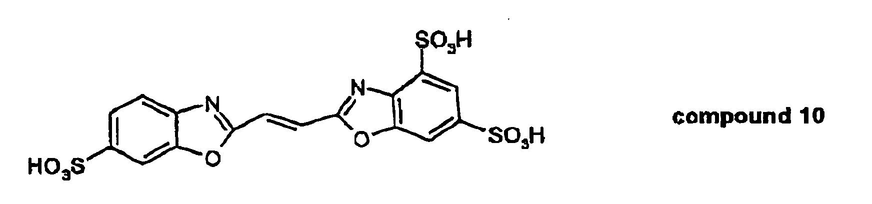

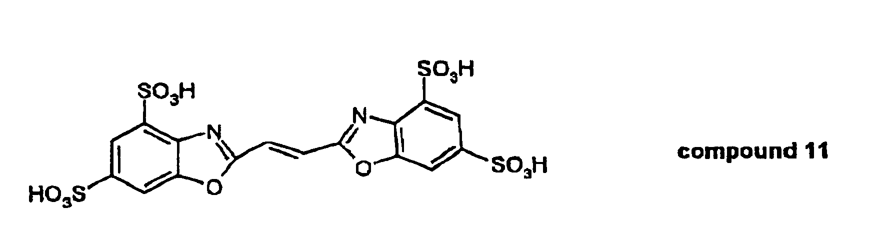

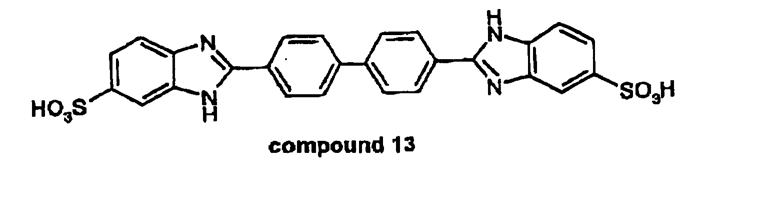

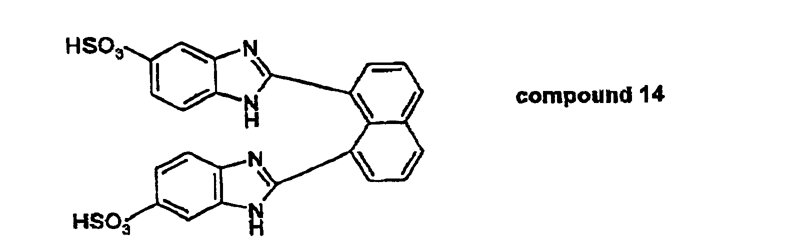

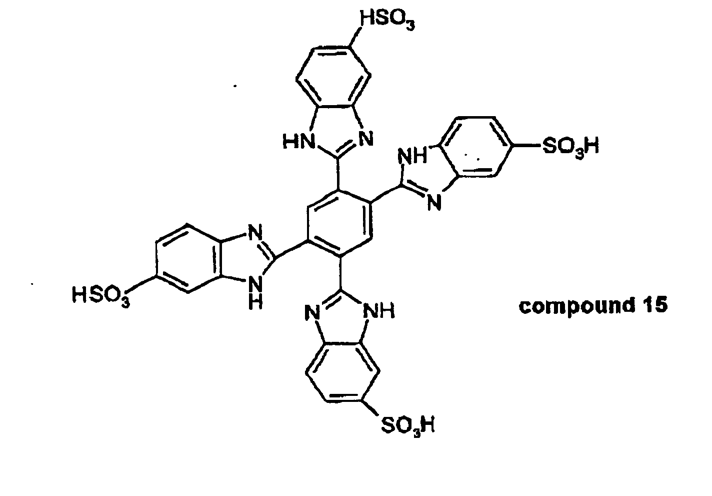

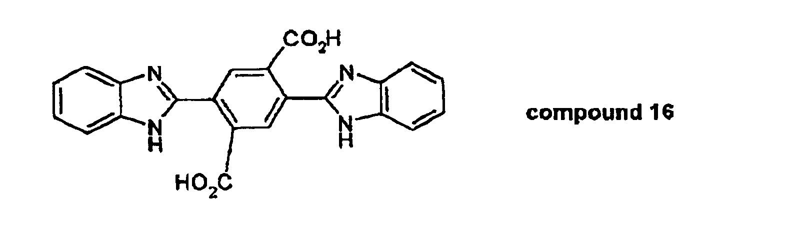

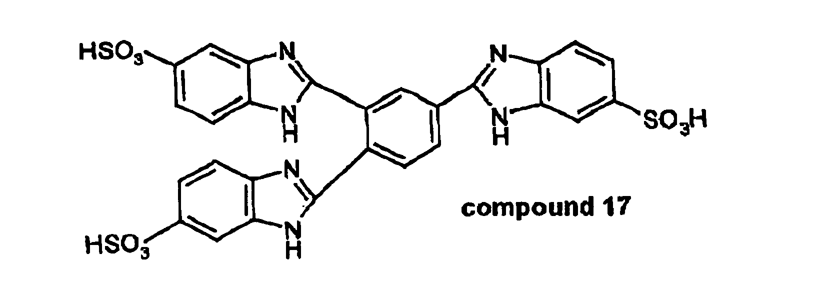

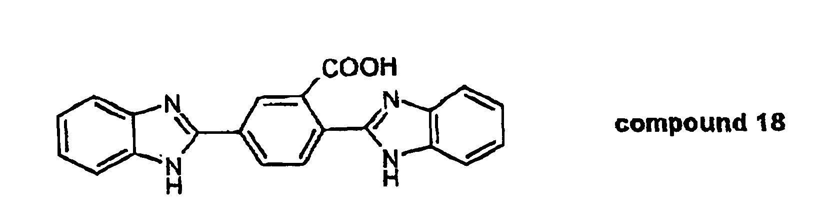

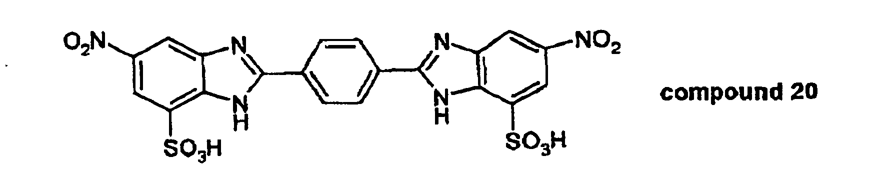

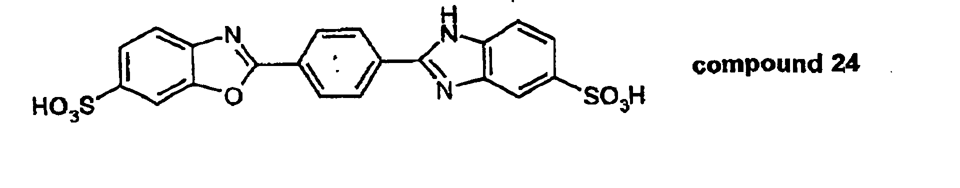

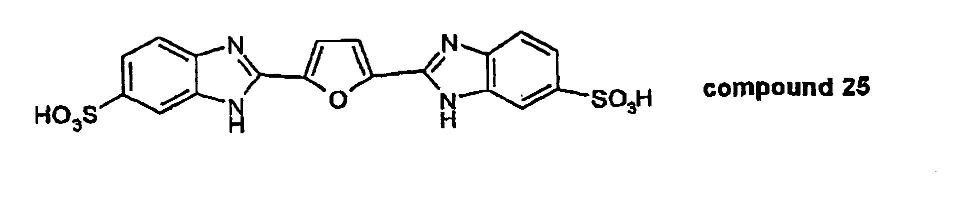

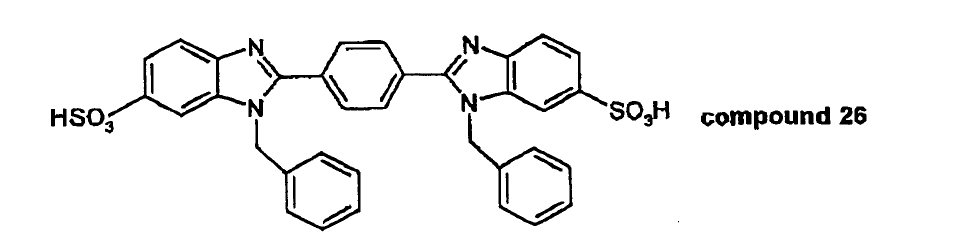

10. Composition according to Claim 9, where the compound of formula (II) is phenylene 1,4-bis benzimidazole-3,3',5,5'-tetra-sulphonic acid or one of its salts with the following structure:

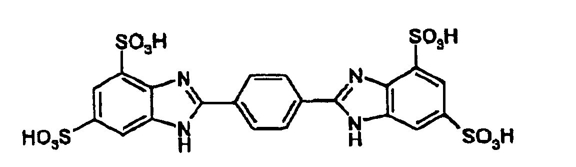

11. composition according to any one of Claims 1 to 10, **characterized in that** the compound or compounds of formula (II) as defined in any one of Claims 1 and 3 to 10 or else the compound or compounds as defined in Claim 2 are present at a concentration of between 0.1 and 15% by weight with respect to the total weight of the composition.

12. Composition according to Claim 11, **characterized in that** the said concentration is between 0.2 and 10% by weight with respect to the total weight of the composition.

13. Composition according to any one of Claims 1 to 12, **characterized in that** the said cosmetically acceptable vehicle is provided in the form of an emulsion of oil-in-water type.

14. Composition according to any one of Claims 1 to 13, **characterized in that** it furthermore comprises one or more additional hydrophilic or lipophilic organic screening agents which are active in the UV-A and/or UV-B regions, other than the said first and second screening agents.

15. Composition according to Claim 14, **characterized in that** the said additional organic screening agents are chosen from cinnamic derivatives, salicylic derivatives, camphor derivatives, triazine derivatives other than those defined in the preceding claims, benzimidazole derivatives other than those defined in the preceding claims, benzophenone derivatives, dibenzoylmethane derivatives, β,β-diphenyl-acrylate derivatives, p-aminobenzoic acid derivatives, or screening polymers and screening silicones.

16. Composition according to any one of Claims 1 to 15, **characterized in that** it furthermore comprises, as additional UV photoprotective agents, pigments or nanopigments formed of metal oxides which are coated or non-coated.

17. Composition according to Claim 16, **characterized in that** the said pigments or nanopigments are chosen from titanium, zinc, iron, zirconium or cerium oxides and their mixtures which are coated or non-coated.

18. Composition according to any one of Claims 1 to 17, **characterized in that** it additionally comprises at least one agent for the artificial tanning and/or browning of the skin.

19. Composition according to any one of Claims 1 to 18, **characterized in that** it additionally comprises at least one adjuvant chosen from fatty substances, organic solvents, thickeners, softeners, antioxidants, opacifiers, stabilizers, emollients, hydroxy acids, antifoaming agents, moisturizing agents, vitamins, fragrances, preservatives, surfactants, fillers, sequestering agents, polymers, propellants, basifying or acidifying agents, or dyes.

20. Composition according to any one of Claims 1 to 19, **characterized in that** it is a composition for the protection of the human epidermis or an antisun composition and **in that** the composition is provided in the form of a non-ionic vesicular dispersion, of an emulsion, in particular of an emulsion of oil-in-water type, of a cream, of a milk, of a gel, of a cream gel, of a suspension, of a dispersion, of a powder, of a solid tube, of a foam or of a spray.

21. Composition according to any one of Claims 1 to 20, **characterized in that** it is a composition for making up the eyelashes, eyebrows or skin and **in that** the composition is provided in the anhydrous or aqueous, pasty or solid form of an emulsion, of a suspension or of a dispersion.

22. Composition according to any one of Claims 1 to 20, **characterized in that** it is a composition intended for the protection of the hair against ultraviolet rays and **in that** the composition is provided in the form of a shampoo, of a lotion, of a gel, of an emulsion or of a non-ionic vesicular dispersion.

23. Use of the composition defined according to any one of Claims 1 to 22 in the manufacture of cosmetic compositions for the protection of the skin or hair against ultraviolet radiation, in particular solar radiation.

24. Cosmetic treatment process for protecting the skin and/or hair against ultraviolet radiation, in particular solar radiation, **characterized in that** it consists in applying, to the skin and/or hair, an effective amount of a composition as defined in any one of Claims 1 to 22.
